# EUROPEAN PATENT SPECIFICATION

(11) **EP 0 988 309 B1**
(45) Date of publication and mention of the grant of the patent: **23.11.2005**
(21) Application number: 98919414.7
(22) Date of filing: 25.05.1998
(51) Int. Cl.: C07H 17/08, A61K 31/70

(54) **C-4''-SUBSTITUTED MACROLIDE DERIVATIVES**
C-4"-SUBSTITUTIERTE MACROLID-DERIVATE
DERIVES DE MACROLIDE SUBSTITUE EN C-4''

(30) Priority: 11.06.1997 US 49980 P
(43) Date of publication of application: 29.03.2000
(73) Proprietor: Pfizer Products Inc., Groton, Connecticut 06340 (US)
(72) Inventor: BRONK, Brian, Scott, Gales Ferry, CT 06335 (US); KANEKO, Takushi, Guilford, CT 06437 (US); LETAVIC, Michael, Anthony, Mystic, CT 06355 (US); CHENG, Hengmiao, East Lyme, CT 06333 (US); GLAZER, Edward, Alan, Waterford, CT 06385 (US); YANG, Bingwei, Vera, Waterford, CT 06385 (US)
(74) Representative: Stuart, Peter Graham
(86) International application number: PCT/IB1998/000799
(87) International publication number: WO 1998/056801

(56) References cited:
- EP-A- 0 136 831
- EP-A- 0 508 699
- FR-A- 2 385 735
- US-A- 4 512 982
- US-A- 5 441 939

## Description

This invention relates to novel C-4" substituted macrolide derivates that are useful as antibacterial and antiprotozoa agents in mammals, including man, as well as in fish and birds. This invention also relates to pharmaceutical compositions containing the novel compounds and to methods of treating bacterial and protozoa infections in mammals, fish and birds by administering the novel compounds to mammals, fish and birds requiring such treatment.

Macrolide antibiotics are known to be useful in the treatment of a broad spectrum of bacterial and protozoa infections in mammals, fish and birds. Such antibiotics include various derivatives of erythromycin A such as azithromycin which is commercially available and is referred to in United States patents 4,474,768 and 4,517,359. Like azithromycin and other macrolide antibiotics, the novel macrolide compounds of the present invention possess potent activity against various bacterial and protozoa infections as described below.

United States patent no 5,441,939 discloses erythromycin derivatives wherein the substituent at the C-3" position are methyl and hydrogen.

French patent no 2385735 discloses erythromycin derivatives and matricides wherein the substituent at the C-4" position is hydroxy.

United States patent no 4,512,982 discloses erythromycin derivatives wherein the substituent at the C-4" position is selected from the group consisting of hydrogen and amino.

European patent no 0508699 discloses macrolides wherein the substituents at the C-4" position are either both hydrogen, or one substituent is hydrogen and the other is hydroxy, an acyloxy derivative taken from the group consisting of formyloxy.

The compounds of the present invention provide for different substituents at the C-4" and C-3" position. Compounds of the present invention have methyl and methoxy substituents at the C-3" position. It has been surprisingly found that the compounds of the present invention have improved in vivo efficacy in the survival of mice in a respiratory infection model when compared with the compounds of US patent no 5,441,939.

More specifically, the present invention relates to novel C-4" substituted matricides. The C-4" substituted matricides of the present invention have been surprisingly found to be more effective than the compounds disclosed in French patent no 2385735, European patent nos 0508699 and 0136831 and US patent nos 5,441,939 and 4,512,982 in the treatment of bacterial and protozoal infections in mammals, fish and birds.

### Summary of the Invention

The present invention relates to compounds of the formula 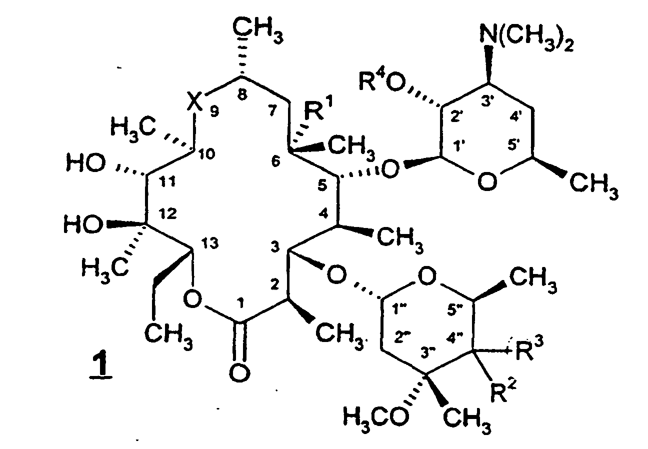 and to pharmaceutically acceptable salts thereof, wherein:
X is -CH(NR⁹R¹⁰)-, -C(O)-, -C(=NOR⁹)-, -CH₂NR⁹-, or -N(C₁-C₆ alkyl)CH₂- wherein the first dash of each of the foregoing X groups is attached to the C-10 carbon of the compound of formula 1 and the last dash of each group is attached to the C-8 carbon of the compound of formula 1;
R¹ is H, hydroxy or methoxy;
R² is hydroxy;
R³ is C₁-C₁₀ alkyl, C₂-C₁₀ alkenyl, C₂-C₁₀ alkynyl, cyano, -CH₂S(O)ₙR⁸ wherein n is an integer ranging from 0 to 2, -CH₂OR⁸, -CH₂N(OR⁹)R⁸, -CH₂NR⁸R¹⁵, -(CH₂)ₘ(C₆-C₁₀ aryl), or -(CH₂)ₘ(5-10 membered heteroaryl), wherein m is an integer ranging from 0 to 4, and wherein the foregoing R³ groups are optionally substituted by 1 to 3 R¹⁶ groups;
or R² and R³ are taken together to form an oxazolyl ring as shown below 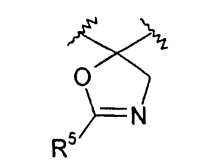 R⁴ is H, -C(O)R⁹, -C(O)OR⁹, -C(O)NR⁹R¹⁰ or a hydroxy protecting group;
R⁵ is -SR⁸, -(CH₂)ₙC(O)R⁸ wherein n is 0 or 1, C₁-C₁₀ alkyl, C₂-C₁₀ alkenyl, C₂-C₁₀ alkynyl, -(CH₂)ₘ(C₆-C₁₀ aryl), or -(CH₂)ₘ(5-10 membered heteroaryl), wherein m is an integer ranging from 0 to 4, and wherein the foregoing R⁵ groups are optionally substituted by 1 to 3 R¹⁶ groups;
each R⁶ and R⁷ is independently H, hydroxy, C₁-C₆ alkoxy, C₁-C₆ alkyl, C₂-C₆ alkenyl, C₂-C₆ alkynyl, -(CH₂)ₘ(C₆-C₁₀ aryl), or -(CH₂)ₘ(5-10 membered heteroaryl), wherein m is an integer ranging from 0 to 4;
each R⁸ is independently H, C₁-C₁₀ alkyl, C₂-C₁₀ alkenyl, C₂-C₁₀ alkynyl, -(CH₂)_{q}CR¹¹R¹²(CH₂)ᵣNR¹³R¹⁴ wherein q and r are each independently an integer ranging from 0 to 3 except q and r are not both 0, -(CH₂)ₘ(C₆-C₁₀ aryl), or -(CH₂)ₘ(5-10 membered heteroaryl), wherein m is an integer ranging from 0 to 4, and wherein the foregoing R⁸ groups, except H, are optionally substituted by 1 to 3 R¹⁶ groups;
or where R⁸ is as -CH₂NR⁸R¹⁵, R¹⁵ and R⁸ may be taken together to form a 4-10 membered saturated monocyclic or polycyclic saturated ring or a 5-10 membered heteroaryl ring, wherein said saturated and heteroaryl rings optionally include 1 or 2 heteroatoms selected from O, S and -N(R⁸)-, in addition to the nitrogen to which R¹⁵ and R⁸ are attached, said saturated ring optionally includes 1 or 2 carbon-carbon double or triple bonds, and said saturated and heteroaryl rings are optionally substituted by 1 to 3 R¹⁶ groups;
each R⁹ and R¹⁰ is independently H or C₁-C₆ alkyl;
each R¹¹, R¹², R¹³ and R¹⁴ is independently selected from H, C₁-C₁₀ alkyl, -(CH₂)ₘ(C₆-C₁₀ aryl), and -(CH₂)ₘ(5-10 membered heteroaryl), wherein m is an integer ranging from 0 to 4, and wherein the foregoing R¹¹, R¹², R¹³ and R¹⁴ groups, except H, are optionally substituted by 1 to 3 R¹⁶ groups;
or R¹¹ and R¹³ are taken together to form -(CH₂)ₚ- wherein p is an integer ranging from 0 to 3 such that a 4-7 membered saturated ring is formed that optionally includes 1 or 2 carbon-carbon double or triple bonds;
or R¹³ and R¹⁴ are taken together to form a 4-10 membered monocyclic or polycyclic saturated ring or a 5-10 membered heteroaryl ring, wherein said saturated and heteroaryl rings optionally include 1 or 2 heteroatoms selected from O, S and -N(R⁸)-, in addition to the nitrogen to which R¹³ and R¹⁴ are attached, said saturated ring optionally includes 1 or 2 carbon-carbon double or triple bonds, and said saturated and heteroaryl rings are optionally substituted by 1 to 3 R¹⁶ groups;
R¹⁵ is H, C₁-C₁₀ alkyl, C₂-C₁₀ alkenyl, or C₂-C₁₀ alkynyl, wherein the foregoing R¹⁵ groups are optionally substituted by 1 to 3 substituents independently selected from halo and -OR⁹;
each R¹⁶ is independently selected from halo, cyano, nitro, trifluoromethyl, azido, -C(O)R¹⁷, -C(O)OR¹⁷, -C(O)OR¹⁷, -OC(O)OR¹⁷, -NR⁶C(O)R⁷, -C(O)NR⁶R⁷, -NR⁶R⁷, hydroxy, C₁-C₆ alkyl, C₁-C₆ alkoxy, -(CH₂)ₘ(C₆-C₁₀ aryl), and -(CH₂)ₘ(5-10 membered heteroaryl), wherein m is an integer ranging from 0 to 4, and wherein said aryl and heteroaryl subsituents are optionally substituted by 1 or 2 substituents independently selected from halo, cyano, nitro, trifluoromethyl, azido, -C(O)R¹⁷, -C(O)OR¹⁷, -C(O)OR¹⁷, -OC(O)OR¹⁷, -NR⁶C(O)R⁷, -C(O)NR⁶R⁷, -NR⁶R⁷, hydroxy, C₁-C₆ alkyl, and C₁-C₆ alkoxy;
each R¹⁷ is independently selected from H, C₁-C₁₀ alkyl, C₂-C₁₀ alkenyl, C₂-C₁₀ alkynyl, -(CH₂)ₘ(C₆-C₁₀ aryl), and -(CH₂)ₘ(5-10 membered heteroaryl), wherein m is an integer ranging from 0 to 4;
with the proviso that R⁸ is not H where R³ is -CH₂S(O)ₙR⁸.

Preferred compounds of formula 1 include those wherein R¹ is hydroxy, R² is hydroxy, R³ is -CH₂NR⁸R¹⁵ or -CH₂SR⁸, and R⁴ is H.

Other preferred compounds of formula 1 include those wherein R¹ is hydroxy, R² is hydroxy, R³ is -CH₂NR⁸R¹⁵, R⁴ is H, R¹⁵ and R⁸ are each selected from H, C₁-C₁₀ alkyl, C₂-C₁₀ alkenyl, and C₂-C₁₀ alkynyl, wherein said R¹⁵ and R⁸ groups, except H, are optionally substituted by 1 or 2 substituents independently selected from hydroxy, halo and C₁-C₈ alkoxy. Specific preferred compounds having the foregoing general structure include those wherein R¹⁵ is either H or is selected from the following groups from which R⁸ is also independently selected: methyl, ethyl, allyl, n-butyl, isobutyl, 2-methoxyethyl, cyclopentyl, 3-methoxypropyl, 3-ethoxypropyl, n-propyl, isopropyl, 2-hydroxyethyl, cyclopropyl, 2,2,2-trifluoroethyl, 2-propynyl, *sec*-butyl, *tert*-butyl, and n-hexyl.

Other preferred compounds of formula 1 include those wherein R¹ is hydroxy, R² is hydroxy, R³ is -CH₂NHR⁸, R⁴ is H, and R⁸ is -(CH₂)ₘ(C₆-C₁₀ aryl) wherein m is an integer ranging from 0 to 4. Specific preferred compounds having the foregoing general structure include those wherein R⁸ is phenyl or benzyl.

Other preferred compounds of formula 1 include those wherein R¹ is hydroxy, R² is hydroxy, R³ is -CH₂NR¹⁵R⁸, R⁴ is H, and R¹⁵ and R⁸ are taken together to form a saturated ring. Specific preferred compounds having the foregoing general structure include those wherein R¹⁵ and R⁸ are taken together to form a piperidino, trimethyleneimino, or morpholino ring.

Other preferred compounds of formula 1 include those wherein R¹ is hydroxy, R² is hydroxy, R³ is -CH₂NR¹⁵R⁸, R⁴ is H, and R¹⁵ and R⁸ are taken together to form a heteroaryl ring optionally substituted by 1 or 2 C₁-C₆ alkyl groups. Specific preferred compounds having the foregoing general structure include those wherein R¹⁵ and R⁸ are taken together to form a pyrrolidino, triazolyl, or imidazolyl ring wherein said heteroaryl groups are optionally substituted by 1 or 2 methyl groups.

Other preferred compounds of formula 1 include those wherein R¹ is hydroxy, R² is hydroxy, R³ is -CH₂SR⁸, R⁴ is H, and R⁸ is selected from C₁-C₁₀ alkyl, C₂-C₁₀ alkenyl, and C₂-C₁₀ alkynyl, wherein said R⁸ groups are optionally substituted by 1 or 2 substituents independently selected from hydroxy, halo and C₁-C₆ alkoxy. Specific preferred compounds having the foregoing general structure include those wherein R⁸ is methyl, ethyl, or 2-hydroxyethyl.

Other preferred compounds of formula 1 include those wherein R¹ is hydroxy, R² is hydroxy, R⁴ is H, and R³ is selected from C₁-C₁₀ alkyl, C₂-C₁₀ alkenyl, and C₂-C₁₀ alkynyl, wherein said R³ groups are optionally substituted by 1 or 2 substituents independently selected from hydroxy, -C(O)R¹⁷, -NR⁶R⁷, halo, cyano, azido, 5-10 membered heteroaryl, and C₁-C₆ alkoxy. Specific preferred compounds having the foregoing general structure include those wherein R³ is methyl, allyl, vinyl, ethynyl, 1-methyl-1-propenyl, 3-methoxy-1-propynyl, 3-dimethylamino-1-propynyl, 2-pyridylethynyl, 1-propynyl, 3-hydroxy-1-propynyl, 3-hydroxy-1-propenyl, 3-hydroxypropyl, 3-methoxy-1-propenyl, 3-methoxypropyl, 1-propynyl, n-butyl, ethyl, propyl, 2-hydroxyethyl, formylmethyl, 6-cyano-1-pentynyl, 3-dimethylamino-1-propenyl, or 3-dimethylaminopropyl.

Other preferred compounds of formula 1 include those wherein R¹ is hydroxy, R² is hydroxy, R⁴ is H, and R³ is -(CH₂)ₘ(5-10 membered heteroaryl) wherein m is an integer ranging from 0 to 4. Specific preferred compounds having the foregoing general structure include those wherein R³ is 2-thienyl, 2-pyridyl, 1-methyl-2-imidazolyl, 2-furyl, or 1-methyl-2-pyrrolyl.

Other preferred compounds of formula 1 include those wherein R¹ is hydroxy, R² is hydroxy, R⁴ is H, and R³ is -(CH₂)ₘ(C₆-C₁₀ aryl) wherein m is an integer ranging from 0 to 4. Specific preferred compounds having the foregoing general structure include those wherein R³ is phenyl.

Specific compounds of formula 1 include those wherein R² and R³ are taken together to form an oxazolyl ring as shown below 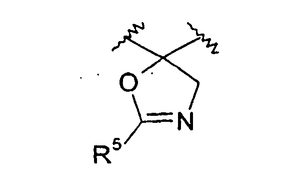 wherein R⁵ is as defined above.

Specific compounds of formula 1 include those wherein R³ is selected from the following: 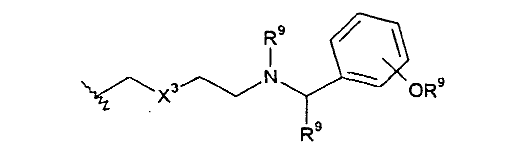 wherein X³ is O, S or -N(R¹⁵)-, and wherein the -OR⁹ group may be attached at any available carbon on the phenyl group.

The invention also relates to a pharmaceutical composition for the treatment of a bacterial infection or a protozoa infection in a mammal, fish, or bird which comprises a therapeutically effective amount of a compound of formula 1, or a pharmaceutically acceptable salt thereof, and a pharmaceutically acceptable carrier.

The invention also relates to a method of treating a bacterial infection or a protozoa infection in a mammal, fish, or bird which comprises administering to said mammal, fish or bird a therapeutically effective amount of a compound of formula 1 or a pharmaceutically acceptable salt thereof.

The term "treatment", as used herein, unless otherwise indicated, includes the treatment or prevention of a bacterial infection or protozoa infection as provided in the method of the present invention.

As used herein, unless otherwise indicated, the terms "bacterial infection(s)" and "protozoa infection(s)" include bacterial infections and protozoa infections that occur in mammals, fish and birds as well as disorders related to bacterial infections and protozoa infections that may be treated or prevented by administering antibiotics such as the compounds of the present invention. Such bacterial infections and protozoa infections, and disorders related to such infections, include the following: pneumonia, otitis media, sinusitus, bronchitis, tonsillitis, and mastoiditis related to infection by *Streptococcus pneumoniae, Haemophilus influenzae, Moraxella catarrhalis, Staphylococcus aureus,* or *Peptostreptococcus* spp.; pharynigitis, rheumatic fever, and glomerulonephritis related to infection by *Streptococcus pyogenes,* Groups C and G streptococci, *Clostridium diptheriae,* or *Actinobacillus haemolyticum;* respiratory tract infections related to infection by *Mycoplasma pneumoniae, Legionella pneumophila, Streptococcus* *pneumoniae, Haemophilus influenzae,* or *Chlamydia pneumoniae;* uncomplicated skin and soft tissue infections, abscesses and osteomyelitis, and puerperal fever related to infection by *Staphylococcus aureus,* coagulase-positive staphylococci (i.e., *S. epidermidis, S. hemolyticus,* etc.), *Streptococcus pyogenes , Streptococcus agalactiae,* Streptococcal groups C-F (minute-colony streptococci), viridans streptococci, *Corynebacterium minutissimum, Clostridium* spp., or. *Bartonella henselae;* uncomplicated acute urinary tract infections related to infection by *Staphylococcus saprophyticus* or *Enterococcus* spp.; urethritis and cervicitis; and sexually transmitted diseases related to infection by *Chlamydia trachomatis, Haemophilus ducreyi, Treponema pallidum, Ureaplasma urealyticum,* or *Neiserria gonorrheae;* toxin diseases related to infection by *S. aureus* (food poisoning and Toxic shock syndrome), or Groups A, B, and C streptococci; ulcers related to infection by *Helicobacter pylori;* systemic febrile syndromes related to infection by *Borrelia recurrentis;* Lyme disease related to infection by *Borrelia burgdorferi*; conjunctivitis, keratitis, and dacrocystitis related to infection by *Chlamydia trachomatis, Neisseria gonorrhoeae, S. aureus, S. pneumoniae, S. pyogenes, H. influenzae,* or *Listeria* spp.; disseminated *Mycobacterium avium* complex (MAC) disease related to infection by *Mycobacterium avium,* or *Mycobacterium intracellulare;* gastroenteritis related to infection by *Campylobacter jejuni;* intestinal protozoa related to infection by *Cryptosporidium* spp.; odontogenic infection related to infection by viridans streptococci; persistent cough related to infection by *Bordetella pertussis;* gas gangrene related to infection by *Clostridium perfringens* or *Bacteroides* spp.; and atherosclerosis related to infection by *Helicobacter pylori* or *Chlamydia pneumoniae.* Bacterial infections and protozoa infections and disorders related to such infections that may be treated or prevented in animals include the following: bovine respiratory disease related to infection by *P. haem., P. multocida, Mycoplasma bovis,* or *Bordetella* spp.; cow enteric disease related to infection by *E. coli* or protozoa (i.e., coccidia, cryptosporidia, etc.); dairy cow mastitis related to infection by *Staph. aureus, Strep. uberis, Strep. agalactiae, Strep. dysgalactiae, Klebsiella* spp., *Corynebacterium,* or *Enterococcus* spp.; swine respiratory disease related to infection by *A. pleuro., P. multocida,* or *Mycoplasma* spp.; swine enteric disease related to infection by *E. coli, Lawsonia intracellularis, Salmonella,* or *Serpulina hyodyisinteriae;* cow footrot related to infection by *Fusobacterium* spp.; cow metritis related to infection by *E. coli;* cow hairy warts related to infection by *Fusobacterium necrophorum* or *Bacteroides nodosus;* cow pink-eye related to infection by *Moraxella bovis;* cow premature abortion related to infection by protozoa (i.e. neosporium); urinary tract infection in dogs and cats related to infection by *E. coli;* skin and soft tissue infections in dogs and cats related to infection by *Staph. epidermidis*, *Staph. intermedius, coagulase neg. Staph.* or *P. multocida;* and dental or mouth infections in dogs and cats related to infection by *Alcaligenes* spp., *Bacteroides* spp., *Clostridium* spp., *Enterobacter* spp., *Eubacterium, Peptostreptococcus, Porphyromonas,* or *Prevotella.* Other bacterial infections and protozoa infections and disorders related to such infections that may be treated or prevented in accord with the method of the present invention are referred to in J. P. Sanford et al., "The Sanford Guide To Antimicrobial Therapy," 26th Edition, (Antimicrobial Therapy, Inc., 1996).

The present invention also relates to a method of preparing the above compound of formula 1, or a pharmaceutically acceptable salt thereof, wherein R³ is -CH₂S(O)ₙR⁸, -CH₂OR⁸ or -CH₂NR⁸R¹⁵, wherein n, R¹⁵ and R⁸ are as defined above with the proviso that R⁸ is not H where R³ is -CH₂S(O)ₙR⁸, which comprises treating a compound of the formula 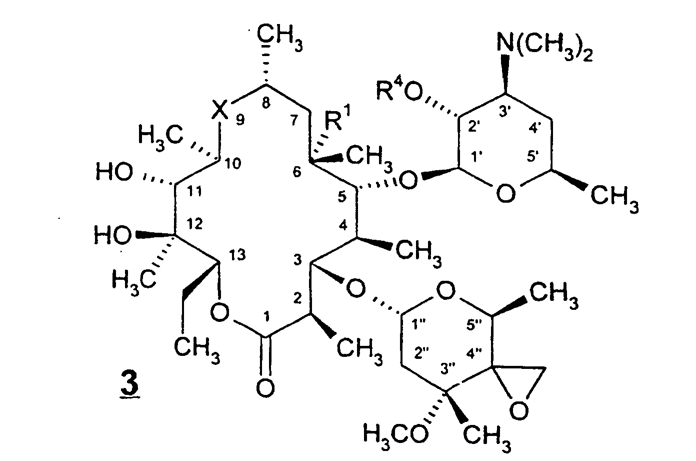 wherein X, R¹ and R⁴ are as defined above, with a compound of the formula HSR⁸, HOR⁸ or HNR¹⁵R⁸, wherein n, R¹⁵ and R⁸ are as defined above, optionally followed by oxidation of the -SR⁸ substituent to form -S(O)R⁸ or -S(O)₂R⁸.

In a further aspect of the above process of preparing the compound of formula 1, or a pharmaceutically acceptable salt thereof, the above compound of formula 3 is prepared by treating a compound of the formula 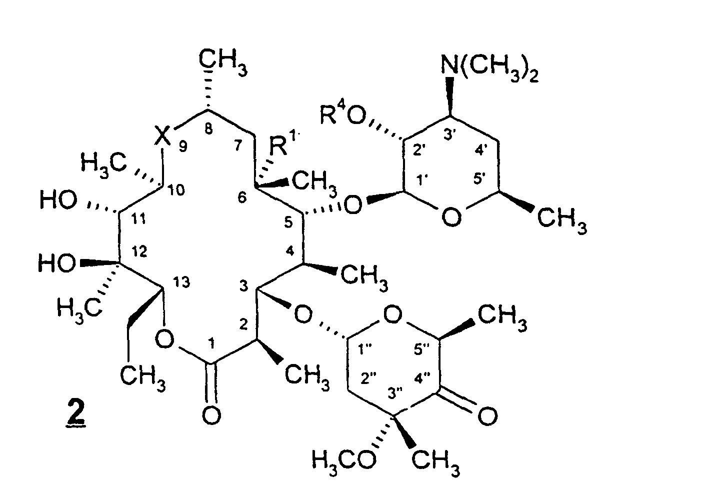 wherein X, R¹ and R⁴ are as defined above, with (CH₃)₃S(O)ₙX² , wherein n is 0 or 1 and X² is halo, -BF₄ or -PF₆, preferably iodo or -BF₄, in the presence of a base such as as potassium tert-butoxide, sodium tert-butoxide, sodium ethoxide, sodium hydride, 1,1,3,3-tetramethylguanidine, 1,8-diazabicyclo[5.4.0]undec-7-ene, 1,5-diazabicylo[4.3.0]non-5-ene, potassium hexamethyldisilazide (KHMDS), potassium ethoxide, or sodium methoxide, preferably KHMDS or a sodium-containing base such as sodium hydride.

The present invention also relates to the above compounds of formulas 2 and 3 which, as indicated above, are useful in the preparation of the above compounds of formula 1 and pharmaceutically acceptable salts thereof.

The term "hydroxy protecting group", as used herein, unless otherwise indicated, includes acetyl, benzyloxycarbonyl, and various hydroxy protecting groups familiar to those skilled in the art including the groups referred to in T. W. Greene, P. G. M. Wuts, "Protective Groups In Organic Synthesis," (J. Wiley & Sons, 1991).

The term "halo", as used herein, unless otherwise indicated, includes fluoro, chloro, bromo or iodo.

The term "alkyl", as used herein, unless otherwise indicated, includes saturated monovalent hydrocarbon radicals having straight, cyclic or branched moieties, or mixtures thereof. It is to be understood that where cyclic moieties are intended, at least three carbons in said alkyl must be present Such cyclic moieties include cyclopropyl, cyclobutyl and cyclopentyl.

The term "alkoxy", as used herein, unless otherwise indicated, includes -O-alkyl groups wherein alkyl is as defined above.

The term "aryl", as used herein, unless otherwise indicated, includes an organic radical derived from an aromatic hydrocarbon by removal of one hydrogen, such as phenyl or naphthyl.

The term "5-10 membered heteroaryl", as used herein, unless otherwise indicated, includes aromatic heterocyclic groups containing one or more heteroatoms each selected from O, S and N, wherein each heterocyclic group has from 5 to 10 atoms in its ring system. Examples of suitable 5-10 membered heteroaryl groups include pyridinyl, imidazolyl, pyrimidinyl, pyrazolyl, (1,2,3,)- and (1,2,4)-triazolyl, pyrazinyl, tetrazolyl, furyl, thienyl, isoxazolyl, oxazolyl, pyrrolyl and thiazolyl.

The phrase "pharmaceutically acceptable salt(s)", as used herein, unless otherwise indicated, includes salts of acidic or basic groups which may be present in the compounds of the present invention. The compounds of the present invention that are basic in nature are capable of forming a wide variety of salts with various inorganic and organic acids. The acids that may be used to prepare pharmaceutically acceptable acid addition salts of such basic compounds of are those that form non-toxic acid addition salts, i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate and pamoate [i.e., 1,1'-methylene-bis-(2-hydroxy-3-naphthoate)] salts. The compounds of the present invention that include an amino moiety may form pharmaceutically acceptable salts with various amino acids, in addition to the acids mentioned above.

Those compounds of the present invention that are acidic in nature are capable of forming base salts with various pharmacologically acceptable cations. Examples of such salts include the alkali metal or alkaline earth metal salts and, particularly, the calcium, magnesium, sodium and potassium salts of the compounds of the present invention.

Certain compounds of the present invention may have asymmetric centers and therefore exist in different enantiomeric and diastereomic forms. This invention relates to the use of all optical isomers and stereoisomers of the compounds of the present invention, and mixtures thereof, and to all pharmaceutical compositions and methods of treatment that may employ or contain them.

The present invention includes the compounds of the present invention, and the pharmaceutically acceptable salts thereof, wherein one or more hydrogen, carbon or other atoms are replaced by isotopes thereof. Such compounds may be useful as research and diagnostic tools in metabolism pharmacokinetic studies and in binding assays.

### Detailed Description of the Invention

The compounds of of the present invention may be prepared according to Schemes 1-3 below and the description that follows. In the following Schemes, unless otherwise indicated, substituents X, R¹, R², R³, R⁴, R⁵, R⁶, R⁷, R⁸, R⁹, R¹⁰, R¹¹, R¹², R¹³, R¹⁴, R¹⁵, R¹⁶ and R¹⁷ are as defined above.

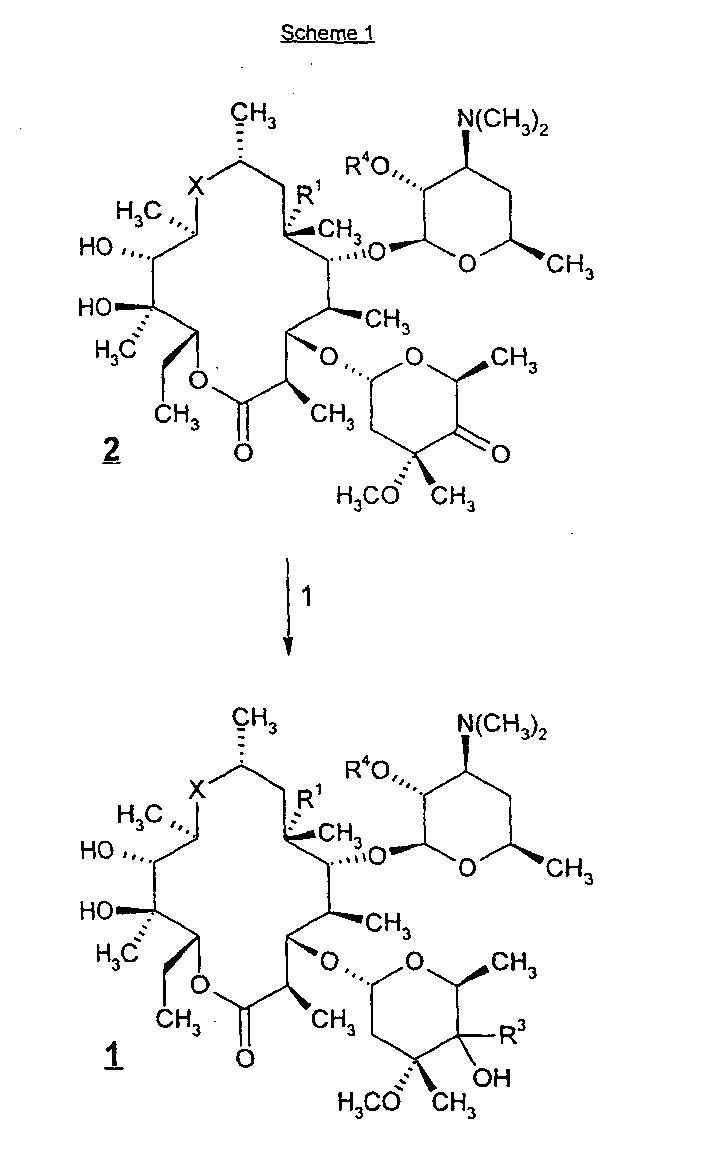

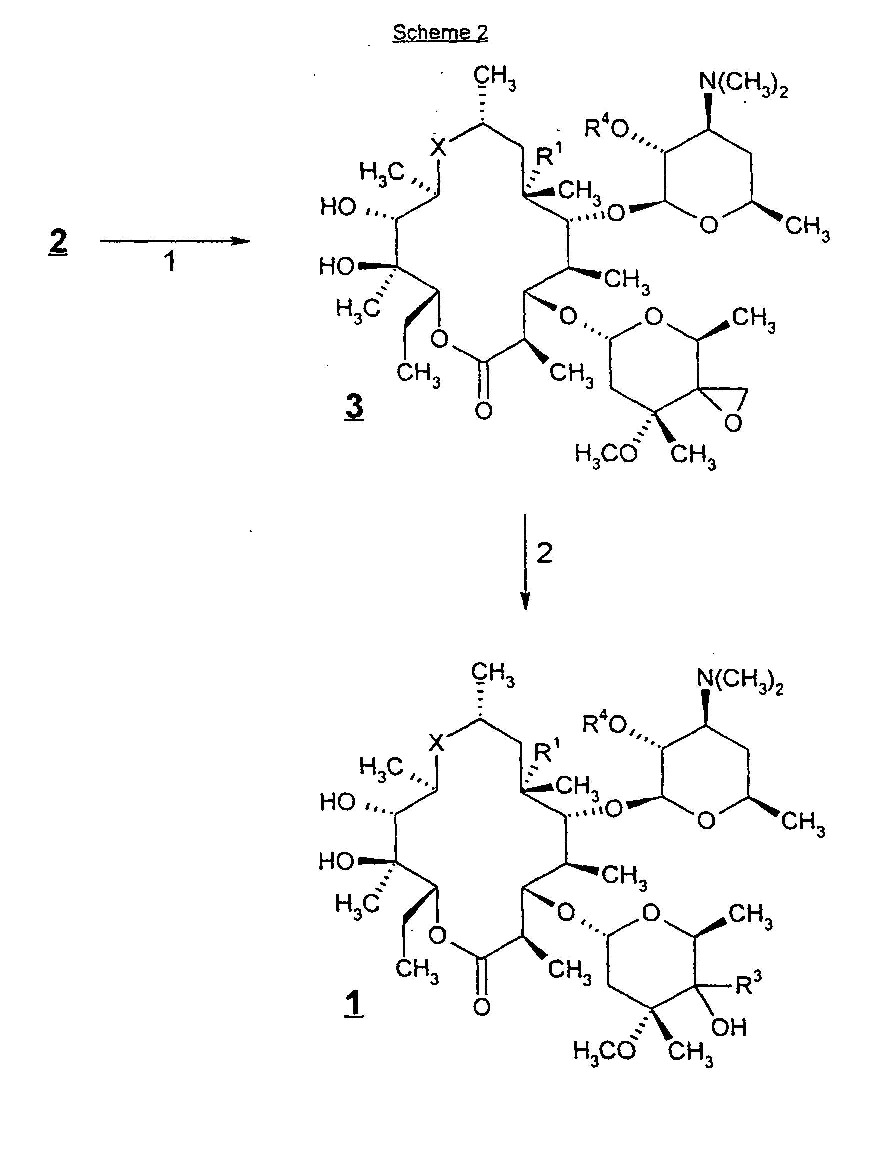

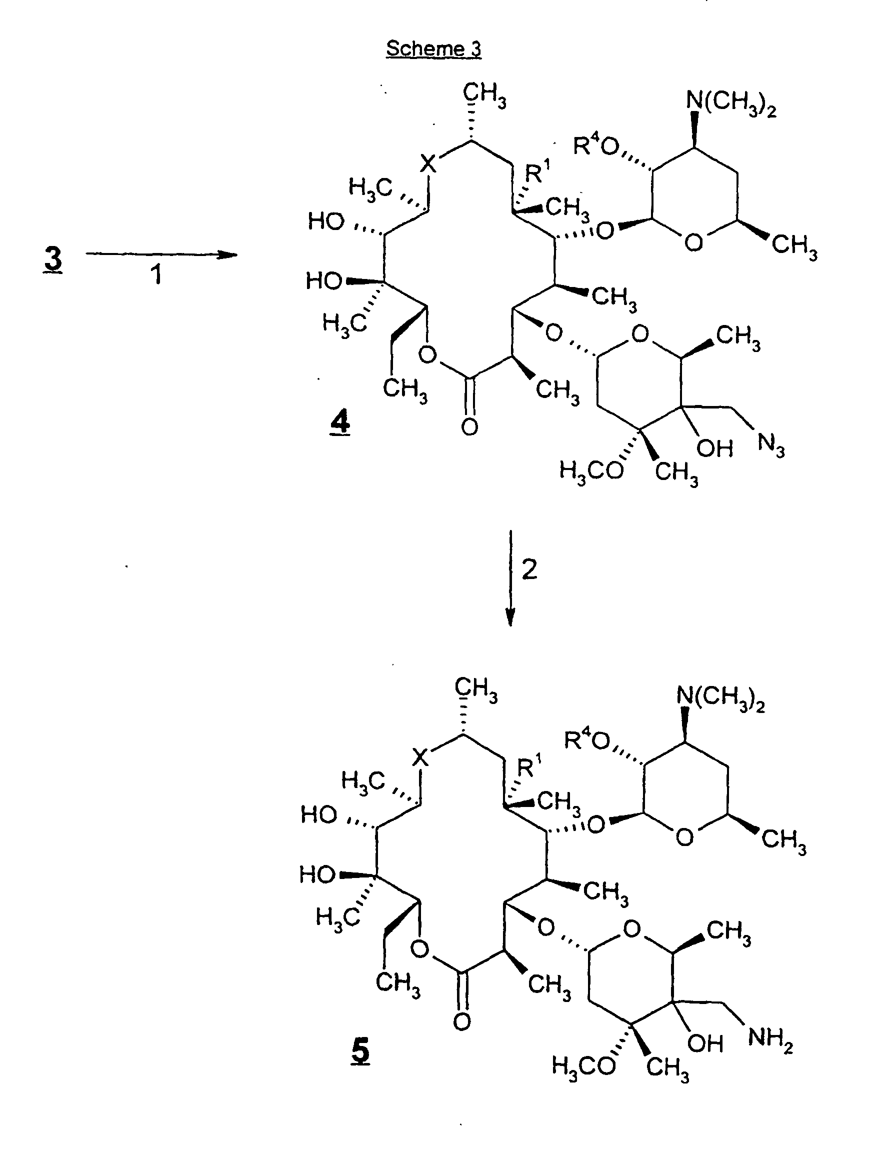

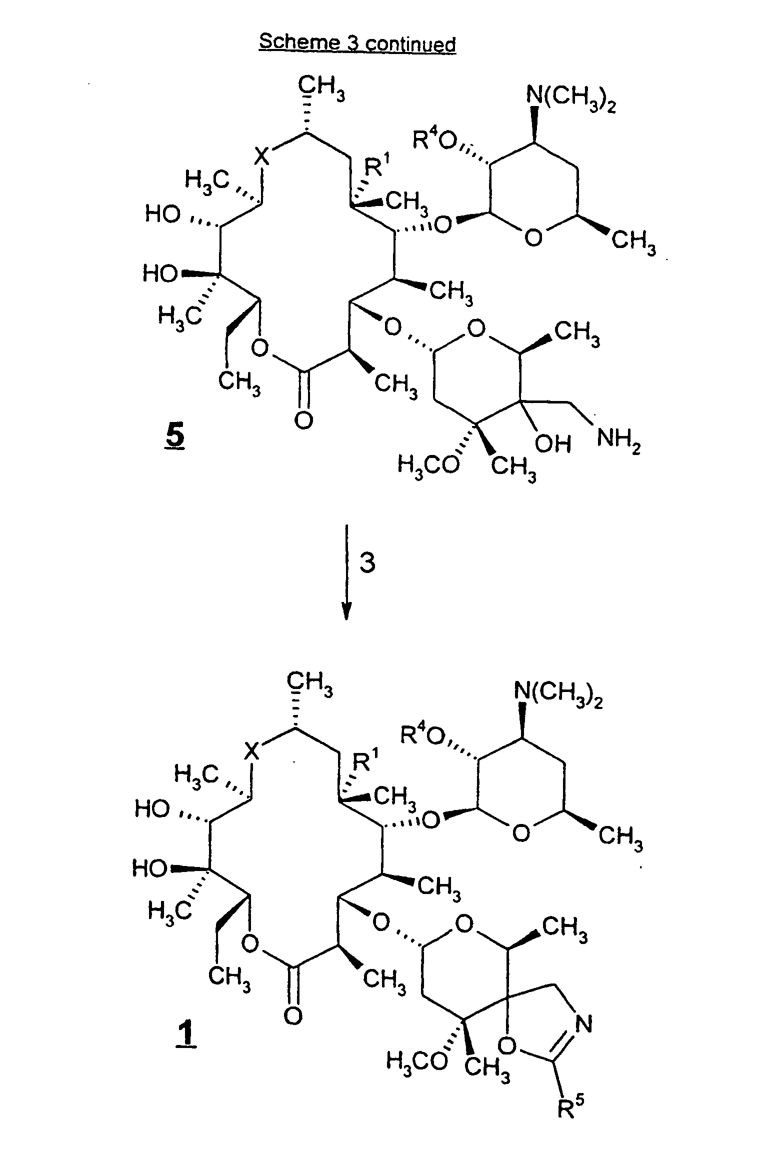

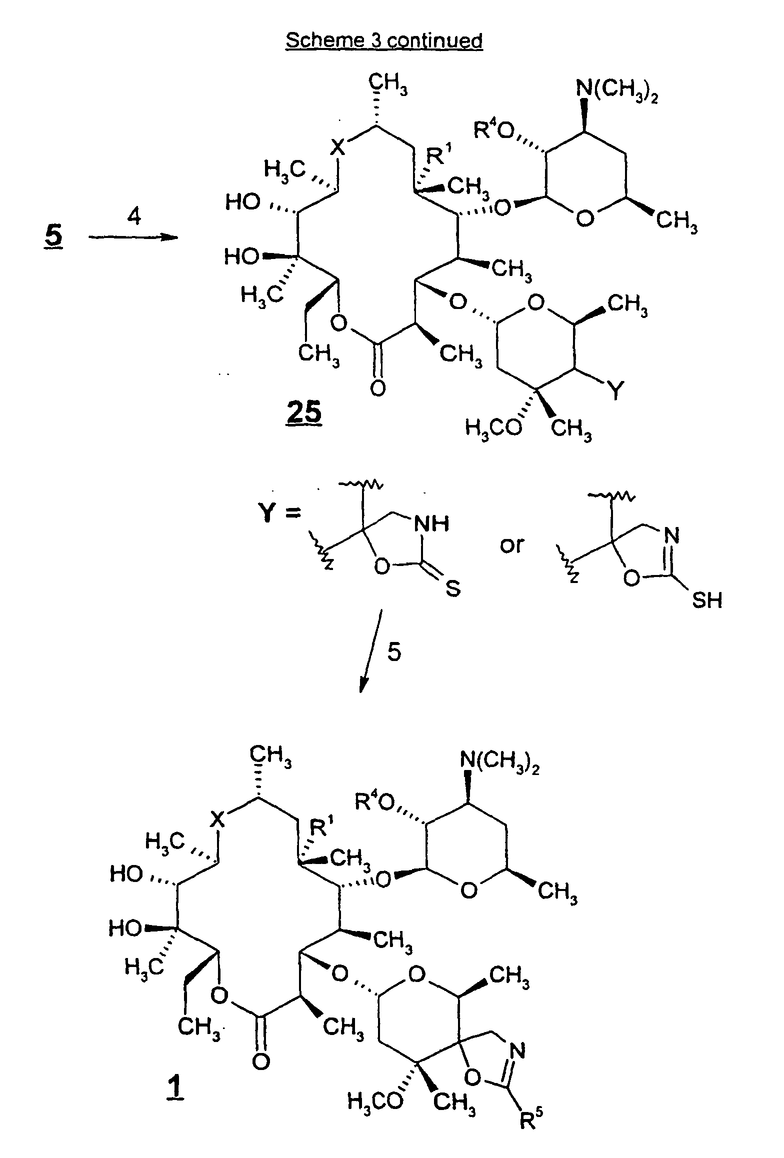

This invention uses a variety of macrolide templates as starting materials. They include azithromycin, erythromycin, clarithromycin, erythromycylamine as well as their analogs. Azithromycin can be prepared according to methods described in United States Patents 4,474,768 and 4,517,359, referred to above. Erythromycin can be prepared, or isolated, according to methods described in United States Patents 2,653,899 and 2,823,203. Clarithromycin can be prepared according to methods described in United States Patent 4,331, 803.

The foregoing starting materials require proper functional group protection before various modifications can take place, and deprotection after desired modifications are complete. The most commonly used protecting groups for amino moieties in the macrolide compounds of this invention are benzyloxycarbonyl (Cbz) and *t*-butyloxycarbonyl (Boc) groups. Hydroxyl groups are generally protected as acetates or Cbz carbonates. The relative reactivity of various hydroxyl groups in the macrolide molecules of the general type claimed in this invention has been well established. Such differences in reactivity permit selective modification of different parts of the compounds of this invention.

In above Schemes, the C-2' hydroxy group (R⁴ is H) is selectively protected by treating the macrolide compound with one equivalent of acetic anhydride in dichloromethane in the absence of external base to provide the corresponding compound wherein R⁴ is acetyl. The acetyl protecting group may be removed by treating the compound of formula 3 with methanol at 23-65°C for 10-48 hours. The C-2' hydroxy may also be protected with other protecting groups familiar to those skilled in the art, such as the Cbz group. Where X is -CH₂NH-, the C-9 amino group may also require protection before further synthetic modifications are performed. Suitable protecting groups for the amino moiety are Cbz and Boc groups. To protect the C-9 amino group, the macrolide may be treated with *t*-butyl dicarbonate in anhydrous tetrahydrofuran (THF) or benzyloxycarbonyl N-hydroxysuccinimide ester or benzylchloroformate to protect the amino group as its *t*-butyl or benzyl carbamate. Both the C-9 amino and C-2' hydroxy may be selectively protected with the Cbz group in one step by treating the compound of formula 2 with benzylchloroformate in THF and water. The Boc group may be removed by acid treatment and the Cbz group may be removed by conventional catalytic hydrogenation. In the following description, it is assumed that, where X is -CH₂NH-, the C-9 amino moiety as well as the C-2' hydroxy group are protected and deprotected as would be deemed appropriate by those skilled in the art.

In Scheme 1, the compound of formula 2 may be prepared according to methods familiar to those skilled in the art, including one or more methods described in the Journal of Antibiotics, 1988, pages 1029-1047. In step 1 of Scheme 1, the compound of formula 2 is treated with R³MgX¹ or R³-Li and Mg(X¹)₂, wherein X¹ is a halide such as chloro or bromo, in a solvent such as THF, ethylene glycol dimethyl ether (DME), diisopropyl ether, toluene, diethyl ether, or tetramethylethylenediamine (TMEDA), hexanes, or a mixture of two or more of the foregoing solvents, preferably an ether solvent, at a temperature ranging from about -78°C to about room temperature (20-25°C), to provide the compound of formula 1 wherein R² is hydroxy and R¹, R³ and R⁴ are as defined above.

Scheme 2 illustrates the preparation of compounds of formula 1 through use of an epoxide intermediate. In step 1 of Scheme 2, the compound of formula 3 may be generated by two methods. In one method (Method A), the compound of formula 2 is treated with (CH₃)₃S(O)X², wherein X² is halo, -BF₄ or -PF₆, preferably iodo, in the presence of a base such as as potassium tert-butoxide, sodium ethoxide, sodium tert-butoxide, sodium hydride, 1,1,3,3-tetramethylguanidine, 1,8-diazabicyclo[5.4.0]undec-7-ene, 1,5-diazabicylo[4.3.0]non-5-ene, potassium ethoxide, or sodium methoxide, preferably a sodium-containing base such as sodium hydride, in a solvent such as THF, an ether solvent, dimethylformamide (DMF), or methyl sulfoxide (DMSO), or a mixture of two or more of the foregoing solvents, at a temperature within the range of about 0°C to about 60°C, to provide the compound of formula 3 in which the following configuration of the epoxide moiety predominates

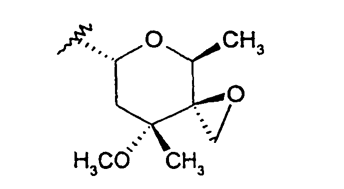

In a second method (Method B), the compound of formula 2 is treated with (CH₃)₃SX², wherein X² is halo, -BF₄ or -PF₆, preferably -BF₄, in the presence of a base such as as potassium tert-butoxide, sodium tert-butoxide, sodium ethoxide, sodium hydride, 1,1,3,3-tetramethylguanidine, 1,8-diazabicyclo[5.4.0]undec-7-ene, 1,5-diazabicylo[4.3.0]non-5-ene, potassium ethoxide, potassium hexamethyldisilazide (KHMDS) or sodium methoxide, preferably KHMDS, in a solvent such as THF, an ether solvent, DMF, or DMSO, or a mixture of two or more of the foregoing solvents, at a temperature within the range of about 0°C to about 60°C, to provide the compound of formula 3 in which the following configuration of the epoxide moiety predominates

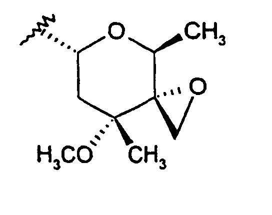

In step 2 of Scheme 2, the compound of formula 3 may be converted to a compound of formula 1 wherein R² is hydroxy and R³ is a group that is attached to the C-4" carbon through a methylene group, such as where R³ is -CH₂NR¹⁵R⁸ or -CH₂S(O)ₙR⁸ wherein n, R¹⁵ and R⁸ are as defined above. To prepare a compound of formula 1 wherein R³ is -CH₂NR¹⁵R⁸, the compound of formula 3 may be treated with a compound of the formula HNR¹⁵R⁸, wherein R¹⁵ and R⁸ are as defined above, in the absence or presence of a polar solvent such as water, methanol, or THF, or a mixture of the foregoing solvents, at a temperature ranging from about room temperature to about 100°C, preferably about 60°C, optionally in the presence of a halide reagent such as potassium iodide, lithium perchlorate, magnesium perchlorate, lithium tetrafluoroborate, pyridinium hydrochloride, or a tetraalkylammonium halide reagent such as tetrabutylammonium iodide. To prepare a compound of formula 1 wherein R³ is -CH₂S(O)ₙR⁸ wherein n and R⁸ are as defined above, the compound of formula 3 may be treated with a compound of the formula HSR⁸ in the presence of K₂CO₃, Kl, or sodium methoxide, in an aromatic solvent such as methanol, benzene or toluene at a temperature ranging from about room temperature to about 120°C. As appropriate, the sulfur moiety may be oxidized to -SO- or -SO₂- according to methods familiar to those skilled in the art. To prepare a compound of formula 1 wherein R³ is -CH₂SR⁸ and R⁸ is -(CH₂)_{q}CR¹¹R¹²(CH₂)ᵣNR¹³R¹⁴, wherein the substituents of said R⁸ group are as defined above, the compound of formula 3 may be treated with a compound of the formula HS-(CH₂)_{q}CR¹¹R¹²(CH₂)ᵣ-NPhth, wherein NPhth represents phthalimido, and potassium iodide to provide the compound of formula 1 wherein R³ is -CH₂S(CH₂)_{q}CR¹¹R¹²(CH₂)ᵣNH₂, after removal of the phthalimido moiety, which may be further modified as necessary. By an analogous method, a compound of formula 1 wherein R³ is -CH₂NR¹⁵R⁸ and R⁸ is -(CH₂)_{q}CR¹³R¹²(CH₂)ᵣNR¹³R¹⁴ may be prepared by treating the compound of formula 3 with either a compound of the formula HNR⁹-(CH₂)_{q}CR¹¹R¹²(CH₂)ᵣ-NR¹³R¹⁴ or a compound of the formula H₂N-(CH₂)_{q}CR¹¹R¹²(CH₂)ᵣ-NH₂ followed by reductive alkylation of the nitrogen atoms. Using the same or an analogous method, a compound of formula 1 wherein R³ is -CH₂OR⁸ and R⁸ is as defined above may be prepared by treating a compound of formula 3 with a compound of the formula HOR⁸.

Scheme 3 illustrates the preparation of compounds of formula 1 in which R² and R³ are taken together to form an oxazolyl moiety. In step 1 of Scheme 3, the compound of formula 3 is treated with sodium azide in the presence of NH₄Cl in methanol or water, or a mixture of the two solvents, at a temperature ranging from about 0°C to about 100°C, preferably about 80°C, to provide the compound of formula 4. In step 2 of Scheme 3, the compound of formula 4 may be converted to the corresponding amine of formula 5 via conventional catalytic hydrogenation. Preferably, such hydrogenation is done using Pd (10% on carbon) powder under an H₂ atmosphere (1 atm). The resulting amine of formula 5 may be converted to various compounds of formula 1 wherein R³ is -CH₂NR¹⁵R⁸ using conventional synthetic methods such as reductive amination.

• In step 3 of Scheme 3, the compound of formula 5 may be converted to the compound of formula 1 wherein R² and R³ are taken together as shown by treating the compound of formula 5 with a compound of formula R⁵-CN, R⁵-C=N(OCH₃), R⁵C=N(OC₂H₅), R⁵-C(O)Cl, or R⁵-CO₂H, wherein R⁵ is as defined above, except it is not NH₂, in the presence or absence of an acid, such as Hcl, or a Lewis acid, such as ZnCl₂ or BF₄Et₃O, or a base, such as NaOH or TEA, in a solvent such as THF, a chlorohydrocarbon (such as CH₂Cl₂ or chlorobenzene), at a temperature ranging from about room temperature to reflux. To prepare the corresponding compound where R⁵ is amino, the compound of formula 5 is treated with BrCN and sodium acetate in methanol at a temperature ranging from about room temperature to reflux. In the alternative, the compound of formula 5 may proceed as indicated in steps 4 and 5 of Scheme 3. In step 4 of Scheme 3, the compound of formula 5 is treated with thiocarbonyldiimidazole in methylene chloride at a temperature ranging from about 0°C to room temperature to provide the compound of formula 25. In step 5 of Scheme 3, the compound of formula 25 is treated with R⁵-X¹, wherein X¹ is a halide such as bromo or iodo, and a base such as sodium methoxide in a solvent such as methanol or acetone at a temperature ranging from about 0°C to room temperature.

The compounds of the present invention may have asymmetric carbon atoms and therefore exist in different enantiomeric and diastereomeric forms. Diastereomeric mixtures can be separated into their individual diastereomers on the basis of their physical chemical differences by methods known to those skilled in the art, for example, by chromatography or fractional crystallization. Enantiomers may be separated by converting the enantiomeric mixtures into a diastereomeric mixture by reaction with an appropriate optically active compound (e.g., alcohol), separating the diastereomers and converting (e.g., hydrolyzing) the individual diastereomers to the corresponding pure enantiomers. Such separations may also be accomplished through use of standard chiral HPLC. The use of all such isomers, including diastereomer mixtures and pure enantiomers, are considered to be part of the present invention.

The compounds of the present invention that are basic in nature are capable of forming a wide variety of different salts with various inorganic and organic acids. Although such salts must be pharmaceutically acceptable for administration to mammals, it is often desirable in practice to initially isolate the compound of the present invention from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained. The desired salt can also be precipitated from a solution of the free base in an organic solvent by adding to the solution an appropriate mineral or organic acid.

Those compounds of the present invention that are acidic in nature are capable of forming base salts with various cations. For compounds that are to be administered to mammals, fish or birds such salts must be pharmaceutically acceptable. Where a pharmaceutically acceptable salt is required, it may be desirable to initially isolate the compound of the present invention from the reaction mixture as a pharmaceutically unacceptable salt and then simply convert the latter to a pharmaceutically acceptable salt in a process analogous to that described above relating to the conversion of pharmaceutically unacceptable acid addition salts to pharmaceutically acceptable salts. Examples of base salts include the alkali metal or alkaline-earth metal salts and particularly the sodium, amine and potassium salts. These salts are all prepared by conventional techniques. The chemical bases which are used as reagents to prepare the pharmaceutically acceptable base salts of this invention are those which form non-toxic base salts with the acidic compounds of the present invention. Such non-toxic base salts include those derived from such pharmacologically acceptable cations as sodium, potassium, calcium, magnesium, various amine cations, etc. These salts can easily be prepared by treating the corresponding acidic compounds with an aqueous solution containing the desired pharmacologically acceptable bases with cations such as sodium, potassium, calcium, magnesium, various amine cations, etc., and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum yields of the desired final product.

The antibacterial and antiprotozoa activity of the compounds of the present invention against bacterial and protozoa pathogens is demonstrated by the compound's ability to inhibit growth of defined strains of human (Assay I) or animal (Assays II and III) pathogens.

### Assay I

Assay I, described below, employs conventional methodology and interpretation criteria and is designed to provide direction for chemical modifications that may lead to compounds that circumvent defined mechanisms of macrolide resistance. In Assay I, a panel of bacterial strains is assembled to include a variety of target pathogenic species, including representatives of macrolide resistance mechanisms that have been characterized. Use of this panel enables the chemical structure/activity relationship to be determined with respect to potency, spectrum of activity, and structural elements or modifications that may be necessary to obviate resistance mechanisms. Bacterial pathogens that comprise the screening panel are shown in the table below. In many cases, both the macrolide-susceptible parent strain and the macrolide-resistant strain derived from it are available to provide a more accurate assessment of the compound's ability to circumvent the resistance mechanism. Strains that contain the gene with the designation of *ermA*/*ermB*/*ermC* are resistant to macrolides, lincosamides, and streptogramin B antibiotics due to modifications (methylation) of 23S rRNA molecules by an Erm methylase, thereby generally prevent the binding of all three structural classes. Two types of macrolide efflux have been described; *msrA* encodes a component of an efflux system in staphylococci that prevents the entry of macrolides and streptogramins while *mefA*/*E* encodes a transmembrane protein that appears to efflux only macrolides. Inactivation of macrolide antibiotics can occur and can be mediated by either a phosphorylation of the 2'-hydroxyl (*mph*) or by cleavage of the macrocyclic lactone (esterase). The strains may be characterized using conventional polymerase chain reaction (PCR) technology and/or by sequencing the resistance determinant. The use of PCR technology in this application is described in J. Sutcliffe et al., "Detection Of Erythromycin-Resistant Determinants By PCR", Antimicrobial Agents and Chemotherapy, 40(11), 2562-2566 (1996). The assay is performed in microtiter trays and interpreted according to Performance Standards for Antimicrobial Disk Susceptibility Tests - Sixth Edition: Approved Standard, published by The National Committee for Clinical Laboratory Standards (NCCLS) guidelines; the minimum inhibitory concentration (MIC) is used to compare strains. Compounds are initially dissolved in dimethylsulfoxide (DMSO) as 40 mg/ml stock solutions.

| Strain Designation | Macrolide Resistance Mechanism(s) |
|---|---|
| *Staphylococcus aureus* 1116 | susceptible parent |
| *Staphylococcus aureus* 1117 | *ermB* |
| *Staphylococcus aureus* 0052 | susceptible parent |
| *Staphylococcus aureus* 1120 | *ermC* |
| *Staphylococcus aureus* 1032 | *msrA, mph,* esterase |
| *Staphylococcus hemolyticus* 1006 | *msrA, mph* |
| *Streptococcus pyogenes* 0203 | susceptible parent |
| *Streptococcus pyogenes* 1079 | *ermB* |
| *Streptococcus pyogenes* 1062 | susceptible parent |
| *Streptococcus pyogenes* 1061 | *ermB* |
| *Streptococcus pyogenes* 1064 | *ermB* |
| *Streptococcus agalactiae* 1024 | susceptible parent |
| *Streptococcus agalactiae* 1023 | *ermB* |
| *Streptococcus pneumoniae* 1016 | susceptible |
| *Streptococcus pneumoniae* 1046 | *ermB* |
| *Streptococcus pneumoniae* 1095 | *ermB* |
| *Streptococcus pneumoniae* 1175 | *mefE* |
| *Streptococcus pneumoniae* 0085 | susceptible |
| *Haemophilus influenzae* 0131 | susceptible |
| *Moraxella catarrhalis* 0040 | susceptible |
| *Moraxella catarrhalis* 1055 | erythromycin intermediate resistance |
| *Escherichia coli* 0266 | susceptible |

Assay II is utilized to test for activity against *Pasteurella multocida* and Assay III is utilized to test for activity against *Pasteurella haemolytica.*

### Assay II

This assay is based on the liquid dilution method in microliter format A single colony of *P. multocida* (strain 59A067) is inoculated into 5 ml of brain heart infusion (BHI) broth. The test compounds are prepared by solubilizing 1 mg of the compound in 125 µl of dimethylsulfoxide (DMSO). Dilutions of the test compound are prepared using uninoculated BHI broth. The concentrations of the test compound used range from 200 µg/ml to 0.098 µg/ml by two-fold serial dilutions. The *P. multocida* inoculated BHI is diluted with uninoculated BHI broth to make a 10⁴ cell suspension per 200 µl. The BHI cell suspensions are mixed with respective serial dilutions of the test compound, and incubated at 37°C for 18 hours. The minimum inhibitory concentration (MIC) is equal to the concentration of the compound exhibiting 100% inhibition of growth of P. multocida as determined by comparison with an uninoculated control.

### Assay III

This assay is based on the agar dilution method using a Steers Replicator. Two to five colonies isolated from an agar plate are inoculated into BHI broth and incubated overnight at 37°C with shaking (200 rpm). The next morning. 300 µl of the fully grown *P. haemolytica* preculture is inoculated into 3 ml of fresh BHI broth and is incubated at 37°C with shaking (200 rpm). The appropriate amounts of the test compounds are dissolved in ethanol and a series of two-fold serial dilutions are prepared. Two ml of the respective serial dilution is mixed with 18 ml of molten BHI agar and solidified. When the inoculated *P. haemolytica* culture reaches 0.5 McFarland standard density, about 5 µl of the *P. haemolytica* culture is inoculated onto BHI agar plates containing the various concentrations of the test compound using a Steers Replicator and incubated for 18 hours at 37°C. Initial concentrations of the test compound range from 100-200 µg/ml. The MIC is equal to the concentration of the test compound exhibiting 100% inhibition of growth of *P. haemolytica* as determined by comparison with an uninoculated control.

The in vivo activity of the compounds of formula (I) can be determined by conventional animal protection studies well known to those skilled in the art, usually carried out in mice.

Mice are allotted to cages (10 per cage) upon their arrival, and allowed to acclimate for a minimum of 48 hours before being used. Animals are inoculated with 0.5 ml of a 3 x 10³ CFU/ml bacterial suspension (*P*. *multocida* strain 59A006) intraperitoneally. Each experiment has at least 3 non-medicated control groups including one infected with 0.1X challenge dose and two infected with 1X challenge dose; a 10X challenge data group may also be used. Generally, all mice in a given study can be challenged within 30-90 minutes, especially if a repeating syringe (such as a Cornwall® syringe) is used to administer the challenge. Thirty minutes after challenging has begun, the first compound treatment is given. It may be necessary for a second person to begin compound dosing if all of the animals have not been challenged at the end of 30 minutes. The routes of administration are subcutaneous or oral doses. Subcutaneous doses are administered into the loose skin in the back of the neck whereas oral doses are given by means of a feeding needle. In both cases, a volume of 0.2 ml is used per mouse. Compounds are administered 30 minutes, 4 hours, and 24 hours after challenge. A control compound of known efficacy administered by the same route is included in each test. Animals are observed daily, and the number of survivors in each group is recorded. The *P. multocida* model monitoring continues for 96 hours (four days) post challenge.

The PD₅₀ is a calculated dose at which the compound tested protects 50% of a group of mice from mortality due to the bacterial infection which would be lethal in the absence of drug treatment.

The compounds of formula 1, and the pharmaceutically acceptable salts thereof (hereinafter "the active compounds"), may be adminstered through oral, parenteral, topical, or rectal routes in the treatment of bacterial and protozoa infections. In general, these compounds are most desirably administered in dosages ranging from about 0.2 mg per kg body weight per day (mg/kg/day) to about 200 mg/kg/day in single or divided doses (i.e., from 1 to 4 doses per day), although variations will necessarily occur depending upon the species, weight and condition of the subject being treated and the particular route of administration chosen. However, a dosage level that is in the range of about 4 mg/kg/day to about 50 mg/kg/day is most desirably employed. Variations may nevertheless occur depending upon the species of mammal, fish or bird being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effects, provided that such larger doses are first divided into several small doses for administration throughout the day.

The active compounds may be administered alone or in combination with pharmaceutically acceptable carriers or diluents by the routes previously indicated, and such administration may be carried out in single or multiple doses. More particularly, the active compounds may be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the active compounds are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably com, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral adinistration, the active compound may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of an active compound in either sesame or peanut oil or in aqueous propylene glycol may be employed. The aqueous solutions should be suitably buffered (preferably pH greater than 8) if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques will known to those skilled in the art.

Additionally, it is also possible to administer the active compounds of the present invention topically and this may be done by way of creams, jellies, gels, pastes, patches, ointments and the like, in accordance with standard pharmaceutical practice.

For administration to animals other than humans, such as cattle or domestic animals, the active compounds may be administered in the feed of the animals or orally as a drench composition.

The active compounds may also be adminstered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The active compounds may also be coupled with soluble polymers as targetable drug carriers. Such polymers can include polyvinylpyrrolidone, pyran copolymer, polyhydroxypropylmethacrylamide phenyl, polyhydroxyethylaspartamide-phenol, or polyethyleneoxide-polylysine substituted with palmitoylresidues. Furthermore, the active compounds may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross-linked or amphipathic block copolymers of hydrogels.

The following Examples further illustrate the method and intermediates of the present invention. It is to be understood that the present invention is not limited to the specific details of the Examples provided below.

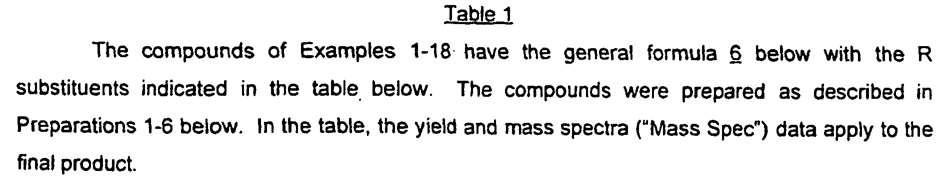

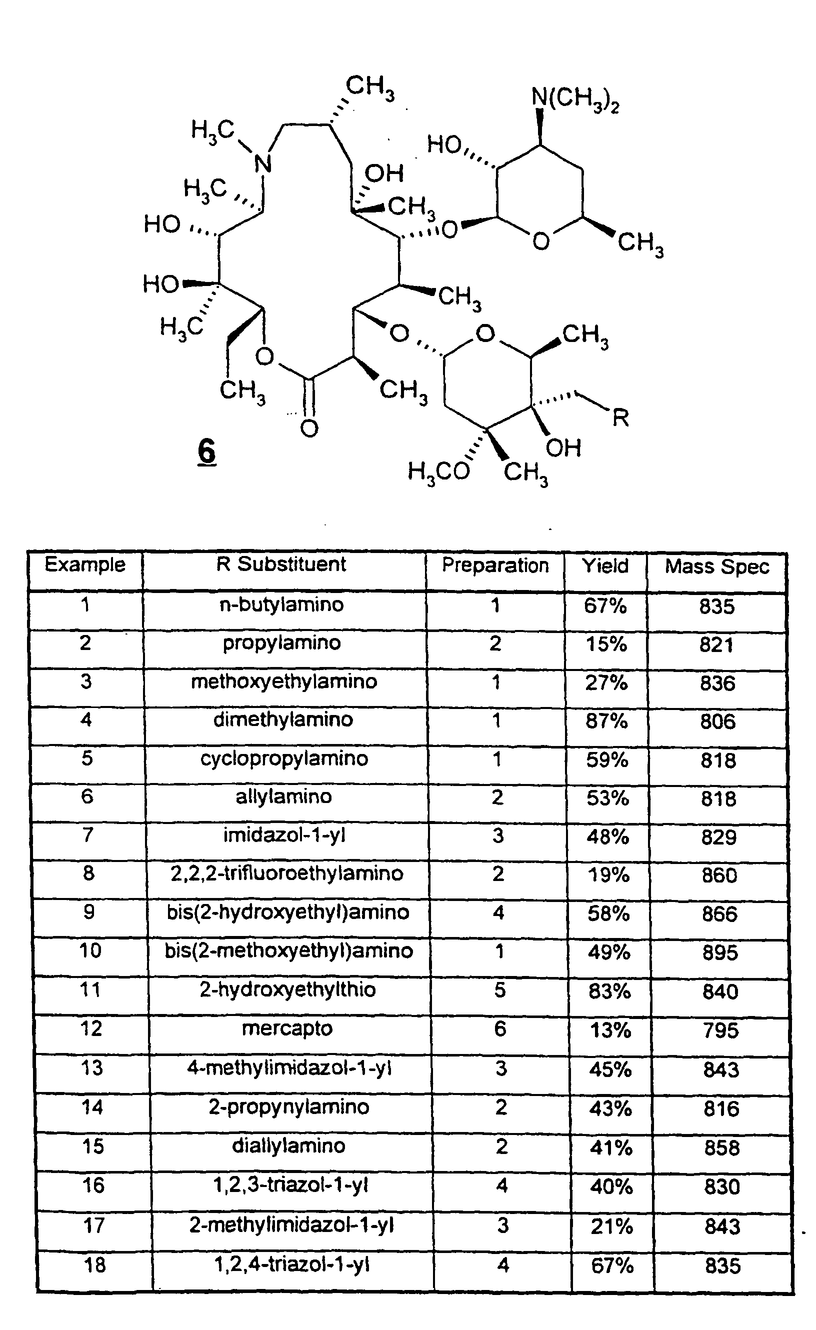

### Preparation methods for Table 1

### Preparation 1

250-500 mg of the compound of formula 3 wherein X is -N(CH₃)CH₂-, R¹ is hydroxy, and R⁴ is H, prepared in accord with Method A referred to above, was dissolved in 1-2 mL of an amine corresponding to the R groups indicated in Table 1 above. A catalytic amount (20 mg) of pyridinium hydrochloride was added and the solution was heated to 50-75°C for approximately two to five days. The reaction was worked up by quenching with 50 mL saturated NaHCO₃. The organic layer was extracted with 3 x 50 mL CH₂Cl₂ and dried over Na₂SO₄. Filtration, concentration of the filtrate, and drying gave a crude oil or solid. Further purification on a silica gel column (1.5-4% MeOH/CHCl₃, 0.2% NH₄OH) afforded the final amino alcohol product.

### Preparation 2

250-500 mg of the compound of formula 3 wherein X is -N(CH₃)CH₂-, R¹ is hydroxy, and R⁴ is H, prepared in accord with Method A referred to above, was dissolved in 1-2 mL of an amine corresponding to the R groups indicated in Table 1 above in a sealed tube. A catalytic amount (20 mg) of pyridinium hydrochloride was added and the solution was heated to 40-75°C for approximately four to eight days. The reaction was worked up by quenching with 50 mL saturated NaHCO₃. The organic layer was extracted with 3 x 50 mL CH₂Cl₂ and dried over Na₂SO₄. Filtration, concentration of the filtrate, and drying gave a crude oil or solid. Further purification on a silica gel column (1.5-4% MeOH/CHCl₃, 0.2% NH₄OH) afforded the final amino alcohol product.

### Preparation 3

300 mg of the compound of formula 3 wherein X is -N(CH₃)CH₂-, R¹ is hydroxy, and R⁴ is H, prepared in accord with Method A referred to above, was dissolved in 2-4 mL MeOH/H₂O. To this was added an imidazole reagent corresponding to the R groups indicated in Table 1 above (25 equiv) and a catalytic amount (20mg) of pyridinium hydrochloride. The reaction mixture was refluxed at 45-50°C for three to four days. The reaction was then quenched with saturated NaHCO₃, extracted with 3 x 300 mL CH₂Cl₂, dried over Na₂SO₄, filtered, and concentrated to a solid. The solid was redissolved in 500 mL EtOAc and washed with 3 x 150 mL 2N NaOH to remove the excess imidazole. Further purification on a silica gel column (2-4% MeOH/CHCl₃, 0.2% NH₄OH) afforded the final product.

### Preparation 4

200-500 mg of the compound of formula 3 wherein X is -N(CH₃)CH₂-, R¹ is hydroxy, and R⁴ is H, prepared in accord with Method A referred to above, was dissolved in 1-2 mL of 2-propanol or methanol. To this was added excess reagent and a catalytic amount (20 mg) of pyridinium hydrochloride. The solution was heated to 40-75°C for approximately two to seven days. The reaction was concentrated down to a crude product. Further purification on a silica gel column (2-4% MeOH/CHCl₃, 0.2% NH₂OH) afforded the final amino alcohol product.

### Preparation 5

180 mg of the compound of formula 3 wherein X is -N(CH₃)CH₂-, R¹ is hydroxy, and R⁴ is H, prepared in accord with Method A referred to above, was dissolved in 2 mL benzene. To this was added excess K₂CO₃ and 0.5 mL of thiol. The mixture was stirred at room temperature for 16 hours. The reaction was quenched with 100 mL saturated NaHCO₃, extracted with 3 x 25 mL CH₂Cl₂, dried over Na₂SO₄, filtered, and concentrated to a solid. Further purification on a silica gel column (2%MeOH/CHCl₃, 0.2% NH₄OH) afforded the final product.

### Preparation 6

115 mg of the compound of formula 3 wherein X is -N(CH₃)CH₂-, R¹ is hydroxy, and R⁴ is H, prepared in accord with Method A referred to above, was dissolved in 3 mL ethanol. To this was added excess thiol. The mixture was heated to 50°C for 4 hours. The reaction was quenched with 100 mL saturated NaHCO₃, extracted with 3 x 25 mL CH₂Cl₂, dried over Na₂SO₄, filtered, and concentrated to a solid. Further purification on a silica gel column (2-4% MeOH/CHCl₃, 0.2% NH₄OH) afforded the final product.

Examples 19-35 below describe the preparation of compounds having the general structure of formula 7 below wherein R is as defined in the examples.

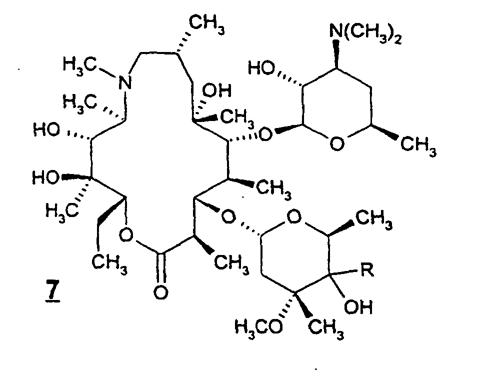

### Example 19

To a solution of methylmagnesium bromide in Et₂O (3.0 M, 1.7 mL) at 0°C was added a solution of methyl propargyl ether (0.421 g, 6 mmol) in THF (5 mL). After stirring at 0°C for 6 hours, a solution of 4"-deoxy-4"-oxo-9-deoxo-9a-aza-9a-methyl-9a-homoerythromycin A (0.224 g, 0.3 mmol) in DME (10 mL) was added at room temperature. After stirring for 1 hour, the reaction mixture was diluted with water (50 mL) and EtOAc (50 mL). After separation, the aqueous layer was washed with EtOAc (3 x 30 mL). The combined organic extracts were washed with a saturated aqueous solution of sodium bicarbonate (40 mL) and brine (40 mL), dried over Na₂SO₄ and concentrated under vacuum. Silica gel chromatography with MeOH - CH₂Cl₂ - NH₄OH (6:93.5:0.5 to 8:91.5:0.5) afforded 0.095 g (39% yield) of the compound of formula 7 wherein R is 3-methoxy-1-propynyl: MS: 817 (API).

### Example 20

To a solution of methylmagnesium bromide in Et₂O (3.0 M, 1.7 mL) at 0°C was added a solution of 1-dimethylamino-2-propyne (0.499 g, 6 mmol) in THF (5 mL). After stirring at 0°C for 6 hours, a solution of 4"-deoxy-4"-oxo-9-deoxo-9a-aza-9a-methyl-9a-homoerythromycin A (0.224 g, 0.3 mmol) in DME (10 mL) was added at room temperature. After stirring at room temperature for 1 hour, the reaction mixture was diluted with water (50 mL) and EtOAc (40 mL). After separation, the aqueous layer was washed with EtOAc (3 x 30 mL). The combined organic extracts were washed with a saturated aqueous solution of sodium bicarbonate (40 mL) and brine (50 mL), dried over Na₂SO₄ and concentrated under vacuum. Silica gel chromatography with MeOH - CH₂Cl₂ - NH₄OH (6:93.5:0.5 to 10:89.5:0.5) afforded 0.093 g (37% yield) of the compound of formula 7 wherein R is 3-dimethylamino-1-propynyl: MS: 831 (API).

### Example 21

To a suspension of trimethylsulfonium tetrafluoroborate (1.03 g, 6.3 mmol) in THF (40 mL) at -10°C was added KHMDS (1.20 g, 6.0 mmol). After stirring below 0°C for 0.5 hour, the reaction vessel was cooled to -78°C and a solution of the compound of formula IV wherein X is -N(CH₃)CH₂- and R¹³ is benzyloxycarboxy (2.60 g, 3 mmol) in DME (10 mL) was added. After 0.5 hour, the reaction mixture was diluted with a saturated aqueous solution of ammonium chloride (40 mL) and EtOAc (50 mL). After separation, the aqueous layer was washed with EtOAc (3 x 30 mL). The combined organic extracts were washed with brine (40 mL), dried over Na₂SO₄ and concentrated under vacuum. Silica gel chromatography with MeOH - CH₂Cl₂ - NH₄OH (2:97.6:0.4 to 4:95.5:0.4) afforded 0.834 g (32% yield) of the compound of formula 3 wherein X is -N(CH₃)CH₂- and R¹³ is benzyloxycarbonyl: MS: 881 (API). The configuration of the epoxide moiety was as provided for Method B relating to Scheme 2 above.

### Example 22

To a solution of the compound of Example 21 (0.101 g, 0.115) in DME (3 mL) was added LiAlH₄ (1.0 M, 2.1 mL) dropwise. After 10 minutes the reaction mixture was treated sequentially with water (0.044 mL), 15% NaOH solution (0.044 mL), and water (0.132 mL), then stirred at rt for 0.5 hour. The mixture was diluted with EtOAc (20 mL) and water (20 mL). After separation the aqueous layer was extracted with EtOAc (3x30 mL). The combined organic extracts were washed with a saturated aqueous solution of sodium bicarbonate (50 mL) and brine (60 mL), dried over Na₂SO₄ and concentrated under vacuum. Silica gel chromatography with MeOH - CH₂Cl₂ - NH₄OH (3:96.5:0.5 to 3.5:95:0.5) afforded 0.042 g (49% yield) of an intermediate compound: MS: 749 (API).

Palladium catalyst (0.075 mg, 10% Pd/C) was added to a solution of the intermediate compound described above (0.151 g. 0.202 mmol) and formaldehyde (0.17 mL, 2.02 mmoL) in methanol (20 mL). The reaction vessel was flushed and filled with hydrogen (50 psi) and shaken at room temperature for 24 hours. The reaction mixture was filtered through Celite™ and concentrated under vacuum. Silica gel chromatography with hexanes - acetone - n-propanol - NH₄OH (100:10:3:0.5 to 50:10:3:0.5) afforded 0.098g (64% yield) of 4"S-methyl-9-deoxo-9a-aza-9a-methyl-9a-homoerythromycin A: MS: 763 (API).

### Example 23

To a solution of 4"-deoxy-4"-oxo-9-deoxo-9a-aza-9a-methyl-9a-homoerythromycin A (1.0 g, 1.34 mmol) in DME (50 mL) at 0°C was added ethynylmagnesium bromide in THF (0.5 M, 40.2 mL). After stirring at 0°C for 0.5 hour the reaction mixture was diluted with a saturated aqueous solution of sodium bicarbonate (100 mL) and EtOAc (100 mL). After separation, the aqueous layer was washed with EtOAc (3 x 100 mL). The combined organic extracts were washed with a saturated aqueous solution of sodium bicarbonate (100 mL) and brine (100 mL), dried over Na₂SO₄ and concentrated under vacuum. Silica gel chromatography with MeOH - CH₂Cl₂ - NH₄OH (4:95.5:0.5) afforded 0.089 g (9% yield) of the compound of formula 7 wherein R is ethynyl: MS: 774 (API).

### Example 24

To a solution of N-methylpyrrole (0.217 g, 2.68 mmol) in THF (5 ml) at -78°C was added BuLi (2.5M, 1.08 ml). The solution was warmed to room temperature over 2 hours and then added via cannula to a flask containing MgCl₂ (0.38 g, 4.02 mmol) and THF (5 mL) at room temperature. After 1 hour at room temperature, a solution of 4"-deoxy-4"-oxo-9-deoxo-9a-aza-9a-methyl-9a-homoerythromycin A (0.200 g, 0.268 mmol) in THF (2 mL) was introduced and stirring was continued at room temperature for 45 minutes. The reaction mixture was diluted with a saturated aqueous solution of sodium bicarbonate (50 mL) and EtOAc (50 mL). After separation, the aqueous layer was washed with EtOAc (3 x 50 mL). The combined organic extracts were washed with a saturated aqueous solution of sodium bicarbonate (50 mL) and brine (50 mL), dried over Na₂SO₄ and concentrated under vacuum. Silica gel chromatography with MeOH - CH₂Cl₂ - NH₄OH (1:98:1 to 8:91:1) afforded 0.032 g (14% yield) of the compound of formula 7 wherein R is 1-methyl-2-pyrrolyl: MS: 829 (API).

### Example 25

To a solution of N-methylimidazole (0.440 g, 5.36 mmol) in THF (5 ml) at -78°C was added BuLi (2.5M, 2.15 ml). The solution was warmed to room temperature over 1 hour and then added via cannula to a flask containing MgCl₂ (0.6374 g, 6.69 mmol) and THF (5 mL) at room temperature. After 2 hours at room temperature, a solution of 4"-deoxy-4"-oxo-9-deoxo-9a-aza-9a-methyl-9a-homoerythromycin A (0.200 g, 0.268 mmol) in DME (2 mL) was introduced and stirring was continued at room temperature for 45 minutes. The reaction mixture was diluted with a saturated aqueous solution of sodium bicarbonate (50 mL) and EtOAc (50 mL). After separation, the aqueous layer was washed with EtOAc (3 x 50 mL). The combined organic - extracts were washed with a saturated aqueous solution of sodium bicarbonate (50 mL) and brine (50 mL), dried over Na₂SO₄ and concentrated under vacuum. Silica gel chromatography with MeOH - CH₂Cl₂ - NH₄OH (1:98:1 to 8:91:1) afforded 0.042 g (19% yield) of the compound of formula 7 wherein R is 1-methyl-2-imidazolyl: MS: 830 (API).

### Example 26

To a solution of an unpurified sample of the compound prepared in Example 20 (0.360 g) in isopropanol (40 mL) was added platinum oxide (0.076 g, 0.335 mmol). The reaction vessel was flushed and filled with hydrogen (50 psi) and shaken at room temperature for 24 hours. Filtration of an aliquot of the reaction mixture through Celite™ and concentration under vacuum afforded the compound of formula 7 wherein R is 3-dimethylamino-1-propenyl: MS: 833 (API).

### Example 27

Platinum oxide (0.076 g, 0.335 mmol) was added to solution remaining from Example 26 and the reaction vessel was flushed and filled with hydrogen (50 psi) and shaken at room temperature for 96 hours. The reaction mixture was filtered through Celite™ and concentrated under vacuum. Silica, gel chromatography with MeOH - CH₂Cl₂ - NH₄OH (1:98:1 to 8:91:1) afforded 0.027 g (5% yield) of the compound of formula 7 wherein R is 3-dimethylaminopropyl: MS: 835 (API).

### Example 28

To a solution of an unpurified sample of the compound prepared in Example 19 (0.400 g) in isopropanol (40 mL) was added platinum oxide (0.076 g, 0.335 mmol). The reaction vessel was flushed and filled with hydrogen (50 psi) and shaken at room temperature for 24 hours. Filtration of an aliquot of the reaction mixture through Celite™ and concentration under vacuum afforded the compound of formula 7 wherein R is 3-methoxy-1-propenyl: MS: 819 (API).

### Example 29

Platinum oxide (0.076 g, 0.335 mmol) was added to solution remaining from Example 26 and the reaction vessel was flushed and filled with hydrogen (50 psi) and shaken at room temperature for 96 hours. The reaction mixture was filtered through Celite and concehtration under vacuum. Silica gel chromatography with MeOH - CH₂Cl₂ - NH₄OH (1:98:1 to 8:91:1) afforded 0.119 g (21% yield) of the compound of formula 7 wherein R is 3-methoxypropyl: MS: 822 (API).

### Example 30

To a flask containing MgB₂•OEt₂ (2.28 g, 8.84 mmol) in DME (5 mL) at 0°C was added propynyllithium (1.865 g, 8.03 mmol). After 6 hours at 0°C, a solution of 4"-deoxy-4"-oxo-9-deoxo-9a-aza-9a-methyl-9a-homoerythromycin A (0.300 g, 0.402 mmol) in DME (2 mL) was introduced and stirring was continued at 0°C for 1 hour, then at room temperature for 0.5 hour. The reaction mixture was diluted with a saturated aqueous solution of sodium bicarbonate (75 mL) and EtOAc (75 mL). After separation, the aqueous layer was washed with EtOAc (3 x 75 mL). The combined organic extracts were washed with a saturated aqueous solution of sodium bicarbonate (75 mL) and brine (75 mL), dried over Na₂SO₄ and concentrated under vacuum. Silica gel chromatography with MeOH - CH₂Cl₂ - NH₄OH (1:98:1 to 8:91:1) afforded 0.099 g (31% yield) of the compound of formula 7 wherein R is 1-propynyl as a mixture of isomers: MS: 788 (API).

### Example 31

To a solution of 4"-deoxy-4"-oxo-9-deoxo-9a-aza-9a-methyl-9a-homoerythromycin A (0.59 g, 0.79 mmol) in THF (20 ml) was added a solution of MeMgBr in Et₂O (1.7 ml, 5.1 mmol, 3.0 M Et₂O solution) at 0°C. The slurry was stirred at 0°C for one hour and was gradually warmed up to room temperature. After 3 hours, the reaction mixture was quenched with a saturated solution of NH₄Cl (10 ml). The organic solvent was removed *in vacuo* on a rotary evaporator. The remaining aqueous solution was adjusted to pH 9.5 with a saturated solution of NaHCO₃ followed by addition of ethyl acetate (30 ml). The aqueous layer, after separation, was extracted with ethyl acetate (2 X 30 ml). The combined organic extracts were washed with brine, dried over magnesium sulfate and concentrated to afford the crude product. Chromatographic purification (silica gel with MeOH/CHCl₃/NH₄OH (4 : 95.9 : 0.1) as eluents), provided 4"R-methyl-9-deoxo-9a-aza-9a-methyl-9a-homoerythromycin A (which .is the compound of formula 7 wherein R is methyl having the R configuration specified) as a white solid, 240 mg (0.315 mmol, 40% yield): FABMS: m/e 763 (MH⁺).

### Example 32

Following the procedure of Example 31, 4"-deoxy-4"-oxo-9-deoxo-9a-aza-9a-methyl-9a-homoerythromycin A (299 mg, 0.403 mmol) and phenyl magnesiumbromide (0.87 ml, 2.61 mmol, 3.0 M THF solution) were reacted to generate the compound of formula 7 wherein R is phenyl, 74 mg (0.09 mmol, 22% yield): FABMS: m/e 825 (MH⁺).

### Example 33

Following the procedure of Example 31, 4"-deoxy-4"-oxo-9-deoxo-9a-aza-9a-methyl-9a-homoerythromycin A (482 mg, 0.646 mmol) and vinyl magnesiumbromide (4.2 ml, 4.2 mmol, 1.0 M THF solution) were reacted to generate the compound of formula 7 wherein R is vinyl, 133 mg (0.172 mmol, 26.6% yield): FABMS: m/e 774 (MH⁺).

### Example 34

Following the procedure of Example 31, 4"-deoxy-4"-oxo-9-deoxo-9a-aza-9a-methyl-9a-homoerythromycin A (494 mg, 0.662 mmol) and benzylmagnesiumchloride (4.4 ml, 4.4 mmol, 1.0 M THF solution) were reacted to generate the compound of formula 7 wherein R is benzyl, 30 mg (0.172 mmol, 5.4% yield): FABMS: m/e 839 (MH⁺).

### Example 35

To a solution of 4"-deoxy-4"-oxo-9-deoxo-9a-aza-9a-methyl-9a-homoerythromycin A (602 mg, 0.806 mmol) in chloroform (8 ml) was added TMSCN (220 ml, 1.64 mmol) followed by Znl₂ (13 mg, 0.04 mmol). The reaction mixture was stirred at room temperature for 30 minutes. A solution of 10% K₂CO₃ in water (10 ml) was added. The organic layer was washed with brine, dried (MgSO₄) and concentrated *in vacuo* to afford the crude product. Chromatography on silica gel with CHCl₃-MeOH-NH₄OH (97 : 3 : 0.1) as eluents afforded the compound of formula 7 wherein R is cyano as a white solid, 94.4 mg (0.122 mmol, 15% yield): FABMS: m/e 774 (MH⁺).

The following scheme illustrates the preparation of the compounds referred to in Table 2 below. In the following scheme, Cbz represents benzyloxycarbonyl.

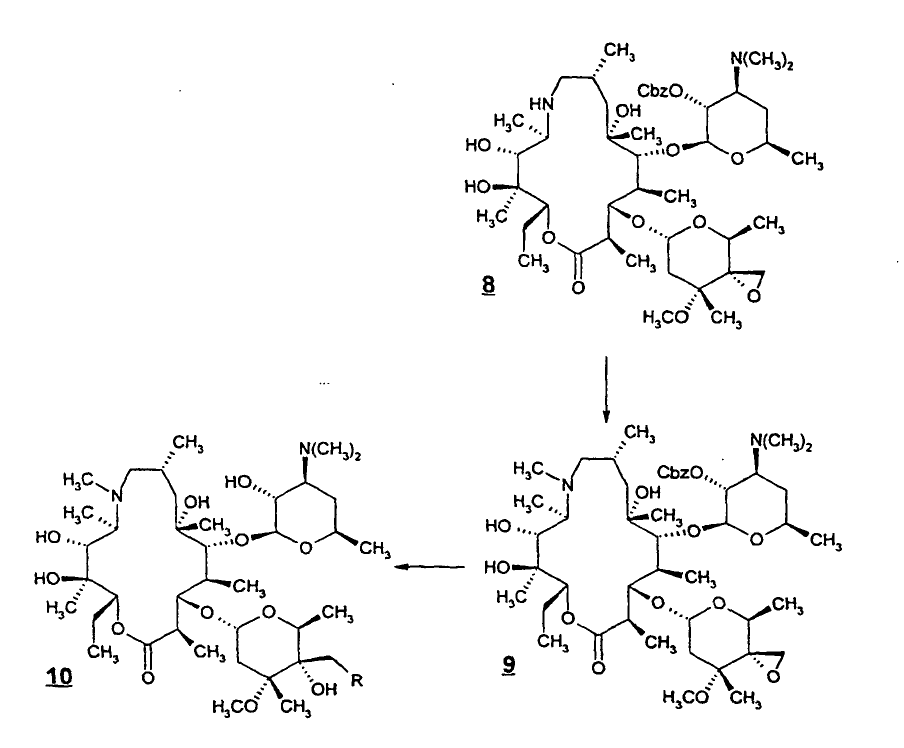

The compound of formula 8, referred to in the scheme above, (20.0g, 22.7 mmol) was dissolved in chloroform (150 mL), followed by the addition of formaldehyde (5.1 mL 37% solution 68.1 mmol) and formic acid (2.8 mL, 74.9 mmol). The resulting solution was heated to 60°C overnight to provide the compound of formula 9. The reaction mixture was poured into water (150 mL) and methylene chloride (50 mL). The organic layer was washed with water (150 mL) one more time, and the aqueous layers were combined, and the pH of the solution was adjusted to 9 by the addition of 5N NaOH solution. The product was then extracted with methylene chloride (3 x 100 mL). The combined organic layers were washed with brine, dried over sodium sulfate, and the organic solvent was removed *in vacuo* to give the compound of formula 9 (19.6 g, 96%). MS (TS) m\z 895.

1-2g of the compound of formula 9 was dissolved in methanol (10 mL), followed by the addition of Kl (10 eq.) and an amine corresponding to the R groups referred to in Table 2 below (10 eq.). After the reaction time indicated below, the reaction mixture was diluted with water (10 mL) and extracted with CH₂Cl₂ (3 x 15 mL). The combined organic layers were washed with brine, dried with Na₂SO₄, filtered and purified by flash chromatography, to provide the compounds of formula 10 with the R groups indicated in Table 2 below.

**Table 2**

| Example | R | Reaction Time (hours) | Yield (%) | Mass Spec |
|---|---|---|---|---|
| 36 | allyamino | 24 | 29 | 818 |
| 37 | propylamino | 48 | 42 | 820 |
| 38 | isopropylamino | 72 | 44 | 820 |
| 39 | cyclopropylamino | 48 | 33 | 818 |
| 40 | isobutylamino | 48 | 43 | 834 |
| 41 | sec-butylamino | 72 | 38 | 834 |
| 42 | dimethylamino | 24 | 35 | 806 |
| 43 | trimethyleneimino | 24 | 30 | 818 |
| 44 | butylamino | 48 | 34 | 834 |
| 45 | diethyamino | 168 | 44 | 834 |
| 46 | ethylamino | 48 | 31 | 806 |
| 47 | *N*-ethylmethylamino | 48 | 36 | 820 |
| 47(a) | pyrrolidino | 96 | 60 | 832.7 |
| 47(b) | piperidino | 96 | 60 | 846.7 |
| 47(c) | 3,4-difluorobenzylamino | 48 | 18.7 | 904.8 |
| 47(d) | 4-methoxybenzylamino | 48 | 17.1 | 898.5 |
| 47(e) | 4-trifluoromethylbenzylamino | 48 | 44.8 | 936.7 |
| 47(f) | anilino | 120 | 31 | 865.7 |
| 47(g) | 4-fluorobenzylamino | 60 | 30 | 886.7 |
| 47(h) | 3-fluorobenzylamino | 48 | 42.8 | 886.7 |
| 47(i) | 2-fluorobenzylamino | 48 | 55.8 | 886.7 |
| 47(j) | 2,4-difluorobenzylamino | 48 | 41.4 | 904.1 |
| 47(k) | 2.5-difluorobenzylamino | 48 | 33.7 | 904.1 |
| 47(l) | 3,5-difluorobenzylamino | 48 | 44.4 | 904.1 |
| 47(m) | 1-(4-fluorophenyl)piperazine | 48 | 25.9 | 941.1 |
| 47(n) | 2-trifluoromethylbenzylamino | 48 | 41.6 | 936.1 |
| 47(o) | 4-trifluoromethoxybenzylamino | 48 | 39.7 | 952.1 |
| 47(p) | 3-trifluoromethoxybenzylamino | 48 | 38.3 | 936.1 |
| 47(q) | 2-fluorophenylethylamino | 48 | 31.2 | 900.2 |
| 47(r) | 3-fluorophenylethylamino | 48 | 25.5 | 900.2 |
| 47(s) | 4-pyridylmethylamino | 48 | 37.9 | 869.6 |
| 47(t) | (methyl)(3-pyridylmethyl)amino | 72 | 11 | 883.5 |
| 47(u) | 4-hydroxy-3-methoxybenzylamino | 48 | 8 | 914.1 |
| 47(v) | piperonylamino | 48 | 25 | 912.1 |
| 47(w) | 3-methoxybenzylamino | 48 | 24 | 898.1 |
| 47(x) | 2-methoxybenzylamino | 48 | 25 | 898.5 |
| 47(y) | 4-fluorophenylethylamino | 48 | 62 | 900.1 |
| 47(z) | 3-pyridylmethylamino | 48 | 30.5 | 869.3 |
| 47(aa) | 2-pyridylmethylamino | 48 | 49.9 | 869.3 |
| 47(ab) | benzylamino | 48 | 28 | 868.6 |

The following scheme illustrates the preparation of compounds referred to in Examples 48-49 below.

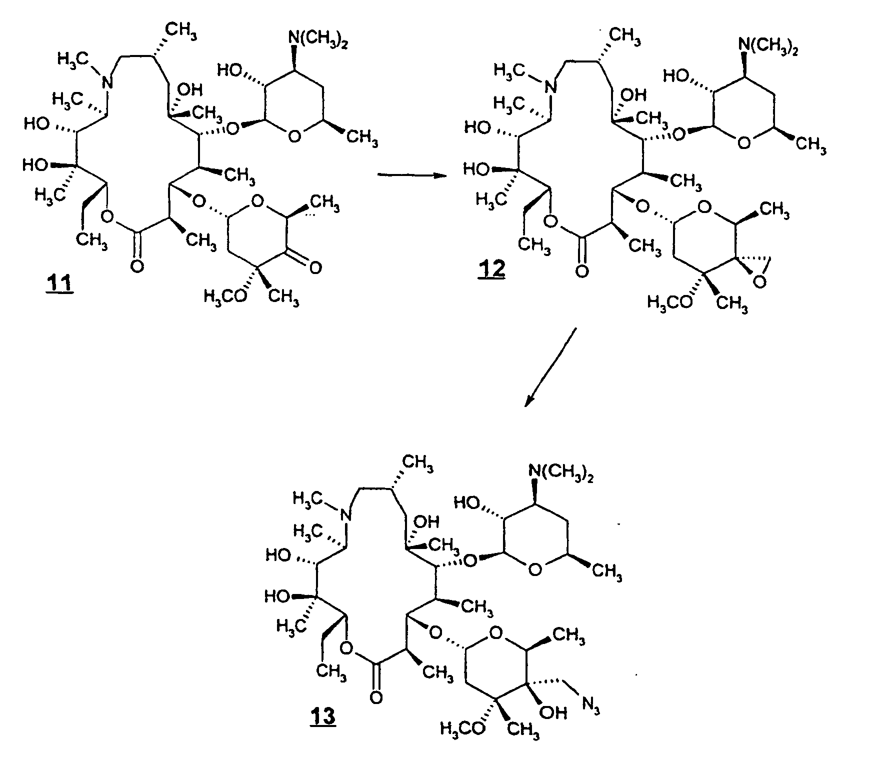

### Example 48

To a solution of sodium hydride (41.5 mg, 1.73 mmol) in DMF (5 ml) was added trimethylsulfoxonium iodide (399 mg. 1.77 mmol). After 15 minutes, the slurry reaction mixture became clear. A solution of 4"-deoxy-4"-oxo-9-deoxo-9a-aza-9a-methyl-9a-homoerythromycin A (940 mg, 1.26 mmol) in DMSO (3 ml) was added slowly. The resulting yellow solution was stirred for 15 minutes at room temperature and 45 minutes at 55°C, and then at room temperature overnight The reaction mixture was taken into water (20 ml) and ethyl acetate (20 ml). The organic layer was washed with brine, dried (MgSO₄) and concentrated to afford the crude product which was chromatographed on silica gel (CHCl₃-MeOH-NH₄OH (97/3/0.1)) to give the above compound of formula 12 as a white solid, 362 mg (0.476 mmol, 38% yield): FABMS: m/e 761 (MH⁺).

### Example 49

To a solution of the compound prepared in Example 48 (95 mg, 0.12 mmol) in 9 ml of MeOH-H₂O (8/1) was added sodium azide (39 mg, 0.60 mmol) followed by NH₄Cl (19 mg, 0.36 mmol). The reaction mixture was heated at 80°C for 24 hours. Methanol was removed *in vacuo* on a rotary evaporator. The product mixture was taken into ethyl acetate (15 ml) and H₂O (15 ml). The aqueous layer, after separation, was extracted with ethyl acetate (15 ml). The combined organic extracts were washed with brine, dried over magnesium sulfate and concentrated to afford the compound of formula 13 as a white solid, 90 mg (0.11 mmol, 93% yield): (FABMS: m/e 804 (MH⁺).

The following scheme illustrates the preparation of compounds referred to in Examples 50-54 below.

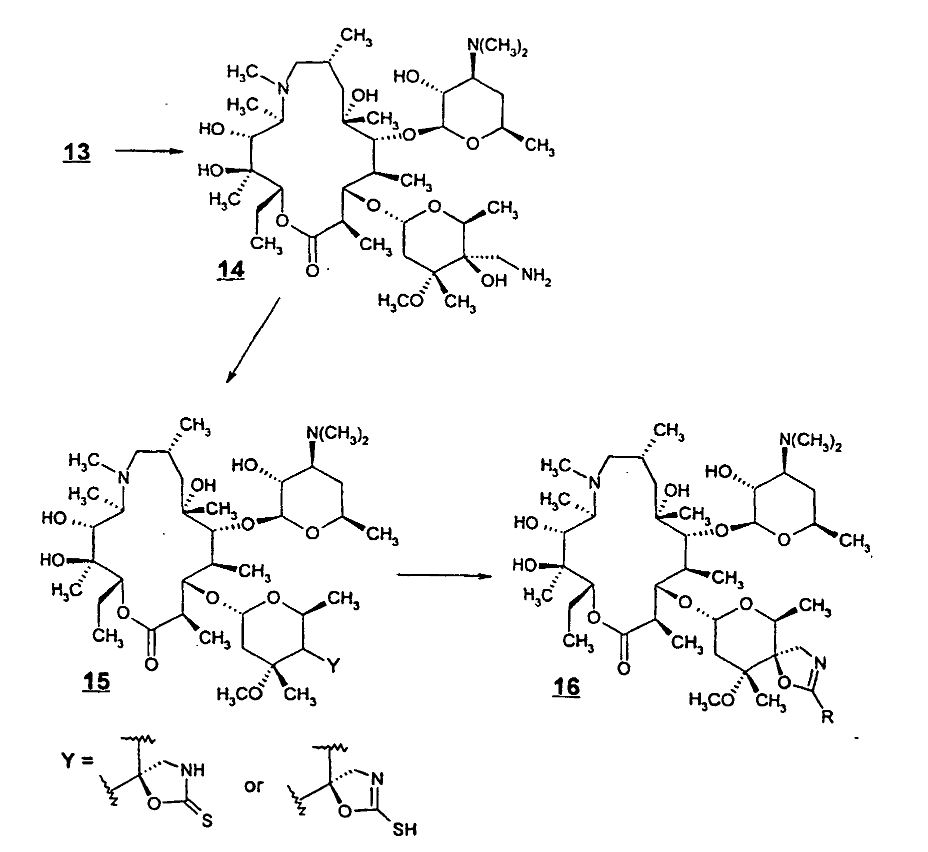

### Example 50

To a solution of the compound prepared in Example 49 (709 mg, 0.882 mmol) was added Pd (10% on carbon) powder (94 mg, 0.088 mmol). The slurry was stirred under H₂ (1 atm) for 18 hours. The reaction mixture was filtered through Celite™. Evaporation of the filtrate afforded the compound of formula 14 as a white solid, 670 mg (0.88 mmol, 100% yield): FABMS: m/e 778 (MH⁺).

### Example 51

To a solution of the compound prepared in Example 50 (163 mg, 0.209 mmol) in CH₂Cl₂ (10 ml) at 0°C was added thiocarbonyldiimidazole (43 mg, 0.242 mmol). The ice bath was removed and the reaction mixture was stirred at ambient temperature overnight. The solvent was removed. The product mixture was taken into ethyl acetate and water. The organic layer was washed with 5% K₂CO₃ solution and then brine, dried over magnesium sulfate and concentrated to afford the compound of formula 15 as a white solid, 170 mg (0.207 mmol, 99% yield).

The compound of formula 15 (168 mg, 0.205 mmol) was dissolved in acetone (6 ml) followed by the addition of 3,4-dichlorophenacyl bromide (63 mg, 0.234 mmol) and sodium bicarbonate (38 mg, 0.417 mmol). The reaction mixture was stirred at ambient temperature for 20 hours The organic solvent was removed. The product mixture was taken into ethyl acetate and was washed with 5% K₂CO₃, brine, dried over magnesium sulfate and concentrated to afford the crude product. Chromatography on silica gel (CHCl₃-MeOH-NH₄OH = 98/2/0.1) gave the compound of formula 16 wherein R is as provided below as a white solid, 90 mg (0.09 mmol, 44% yield): FABMS: m/e 1006 (MH⁺).

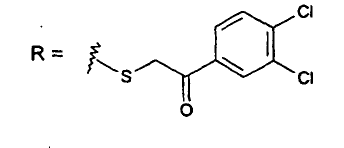

### Example 52

To a solution of the compound of formula 15 (225 mg, 0.274 mmol) in anhydrous methanol (10 ml) was added sodium methoxide (50 mg, 0.926 mmol). The solution was stirred for 10 minutes and cooled to 0°C. Methyl iodide (60 ml, 0.99 mmol) was added dropwise. The reaction mixture was warmed to room temperature and stirred at ambient temperature for 7 hours. The organic solvent was removed. The product mixture was taken into ethyl acetate and was washed with 5% K₂CO₃, brine, dried over magnesium sulfate and concentrated to afford the crude product. Chromatography on silica gel (CHCl₃-MeOH-NH₄OH = 97/3/0.1) gave the compound of formula 16 wherein R is methylthio as a white solid, 231 mg (0.277 mmol, 36% yield): FABMS: m/e 834 (MH⁺).

### Example 53

To a solution of the compound of formula 14 (250 mg, 0.321 mmol) in dichloroethane (10 ml) was added ethyl 2-thiophenecarboximidate hydrochloride (72 mg, 0.461 mmol), which was prepared via bubbling HCl gas through a benzene solution of 2-thiophene carbonitrile and ethanol (1.1 equivalent) for 2 hours and stirring at ambient temperature overnight. The slurry reaction mixture became clear upon addition of triethyl amine (65 ml, 0.467 mmol). It was refluxed overnight. The product mixture was taken into ethyl acetate and water, and the pH was adjusted to 1.9 with 10% HCl solution. The aqueous layer was adjusted to pH 9.5 and extracted with ethyl acetate. The organic extract was washed with brine, dried over magnesium sulfate and concentrated to afford the crude product. Chromatography on silica gel (CHCl₃-MeOH-NH₄OH = 99/1/0.1) gave the compound of formula 16 wherein R is 2-thienyl as a white solid, 92 mg (0.106 mmol, 33% yield): FABMS: m/e 870 (MH⁺).

### Example 54

ZnCl₂ (2 mg) was placed in a round bottom flask and heated to melt under vacuum. After cooled to room temperature, a solution of the compound of formula 14 (236 mg, 0.303 mmol) and 2-cyanopyridine (49 mg, 0.467 mmol) in chlorobenzene (10 ml) was added. The reaction mixture was heated to reflux overnight. Water was added and adjusted to pH 2. After separation, the aqueous layer was adjusted to pH 9.5 and extracted with ethyl acetate. The organic extract was washed with brine, dried over magnesium sulfate and concentrated to afford the crude product. Chromatography on silica gel (CHCl₃-MeOH-NH₄OH = 98/2/0.1) gave the compound of formula 16 wherein R is 2-pyridyl as a white solid, 47 mg (0.054 mmol, 18% yield): FABMS: m/e 865 (MH⁺).

### Example 55

To a solution of the compound of formula 14 (383 mg, 0.492 mmol) in methanol (5 ml) was added a solution of cyanogen bromide (57 mg, 0.538 mmol) and sodium acetate (90 mg, 1.097 mmol) in methanol (5 ml) dropwise. The reaction mixture was stirred at ambient temperature overnight The solvent was evaporated and the solid was taken into ethyl acetate and water, and the pH was adjusted to pH 9.5 with 10% K₂CO₃ solution. The organic extract was washed with brine, dried over magnesium sulfate and concentrated to afford the crude product. Chromatography on silica gel (CHCl₃-MeOH-NH₄OH = 96/4/0.1) gave the compound of formula 16 wherein R is amino as a white solid, 124 mg (0.155 mmol, 31% yield): FABMS: m/e 803 (MH⁺).

The following scheme illustrates the preparation of compounds referred to in Examples 56-63 below.

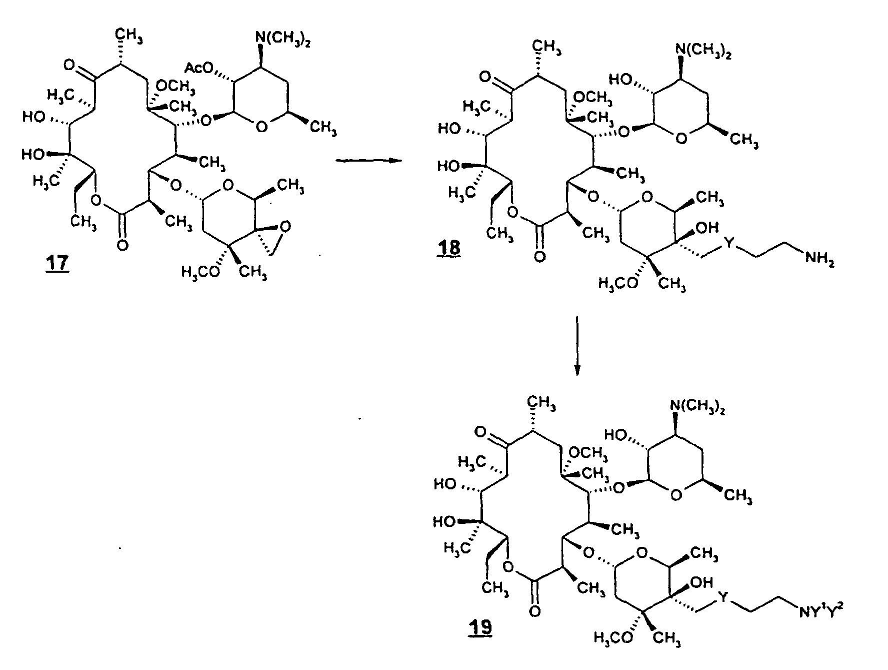

### Example 56

A solution of the compound of formula 17 (3 g, 3.7 mmol) in 30 mL of MeOH was heated at 50°C overnight with 2.25 g (37.5 mmol) of ethylenediamine and 6.21 g (37.1 mmol) of potassium iodide. MeOH was evaporated from the resulting mixture, and the residue was dissolved in CH₂Cl₂ and washed with brine. After drying over Na₂SO₄, CH₂Cl₂ was evaporated under reduced pressure . The residue was chromatographed on SiO₂ (5% MeOH-CH₂Cl₂-0.5% NH₄OH → 10% MeOH-CHCl₂-1%NH₄OH) to give 2.72 g (89%) of the compound of formula 18 wherein Y is -NH-: MS m/e 821 (M+1).

### Example 57

A solution of the compound prepared in Example 56 (1.0 g, 1.2 mmol), *o*-anisaldehyde (174 mg, 1.3 mmol) and sodium acetate (100 mg, 1.2 mmol) in 20 mL of CH₂Cl₂ was stirred at room temperature for 1 hour. To this solution were added 388 mg (1.8 mmol) of sodium triacetoxyborohydride. After 2.5 hour of stirring at room temperature, the reaction mixture was diluted was CH₂Cl₂ and washed with a saturated NaHCO₃ solution and brine. After drying over Na₂SO₄, the organic solvent was removed. The residue was chromatographed twice on SiO₂ (2% MeOH-CH₂Cl₂-0.2% NH₄OH). The material was further purified by preparative SiO₂ plates (10% MeOH-CH₂Cl₂-1% NH₄OH) to give 660 mg (58%) of the compound of formula 19 wherein Y is -NH-, Y¹ is H, and Y² is 2-methoxybenzyl: MS m/e 940 (M+1).

### Examples 58-59

In methods analogous to that of Example 57, by replacing *o*-anisaldehyde with p-trifluoromethylbenzaldehyde and p-phenoxybenzaldehyde the compounds of Examples 58 and 59, respectively, were generated wherein said compounds had the general structure of formula 19 and Y and Y¹ are as defined for the compound of Example 57 and Y² is as provided below.

| Example | Y² | Mass Spec | Yield |
|---|---|---|---|
| 58 | 4-trifluoromethylbenzyl | 978 (M+1) | 33% |
| 59 | 4-phenoxybenzyl | 1002 (M+1) | 46% |

### Example 60

A solution of the compound prepared in Example 57 above (468 mg, 0.5 mmol), isobutyraldehyde (36 mg, 0.5 mmol), and sodium acetate (42 mg, 0.5 mmol) in 5 mL of CH₂Cl₂ was stirred at room temperature for 1.5 hour. To this solution were added 164 mg (0.77 mmol) of sodium triacetoxyborohydride. After stirring at room temperature for 0.5 hr, the reaction mixture was diluted with CH₂Cl₂ and washed with a NaHCO₃ solution and brine. After drying over MgSO₄, the solvent was removed under reduced pressure. The residue was chromatographed on SiO₂ (4% MeOH-CH₂Cl₂-0.4% NH₄OH) to give 256 mg (51%) of the compound of formula 19 wherein Y is -NH-, Y¹ is 2-methylpropyl, and Y² is 2-methoxybenzyl: MS m/e 996 (M+1).

### Example 61

A solution of the compound of formula 20 (522 mg, 0.65 mmol), 2-phthalimidoethanethio (1.08 g, 5.2 mmol) and potassium iodide (865 mg, 5.2 mmol) in 5 mL of MeOH was heated under N₂ for 48 hours. MeOH was then removed under reduced pressure, and the residue was dissolved in CH₂Cl₂ and washed with a NaHCO₃ solution and brine. After drying over MgSO₄, CH₂Cl₂ was removed under reduced pressure. The residue obtained was dissolved in 10 mL of EtOH and treated with 7.5 mL of hydrazine hydrate. After stirring at room temperature for 3 hours EtOH was removed under reduced pressure, and the residue was extracted with CH₂Cl₂. The organic layer was washed with brine and dried over MgSO₄. A SiO₂ chromatography of the residue (4% MeOH- CH₂Cl₂ -0.4% NH₄OH → 5% MeOH-CH₂Cl₂-0.5% mH4OH) gave 287 mg (53%) of the compound of formula 18 wherein Y is S: MS m/e 837 (M+1).

### Example 62

In a method analogous to that of Example 57 and starting with the compound of Example 60, a compound of formula 19 wherein Y is S, Y¹ and Y² are both 2-methoxybenzyl (79% yield, MS m/e 957 (M+1)) and a compound of formula 19 wherein Y is S, Y¹ is H, and Y² is 2-methoxybenzyl (3% yield, MS m/e 1077 (M+1)) were obtained.

### Example 63

In a method analogous to that of Example 60 and starting with the compound of formula 19 wherein Y is S, Y¹ is H, and Y² is 2-methoxybenzyl, and propionaldehyde, the compound of formula 19 wherein Y is S, Y¹ is n-propyl, and Y² is 2-methoxybenzyl was obtained in 70% yield, MS m/e 999 (M+1).

The following scheme illustrates the preparation of compounds referred to in Examples 64- 72 below.

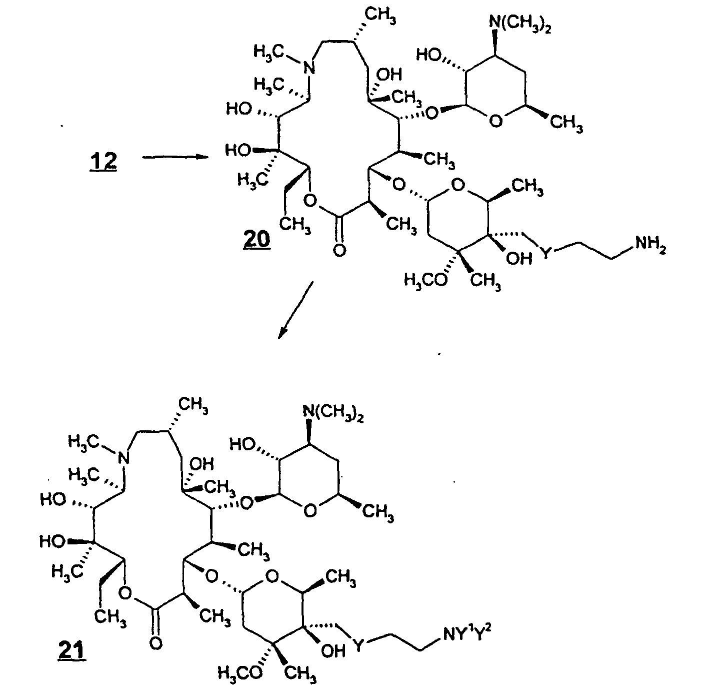

### Example 64

Starting with the compound of formula 12, the compound of formula 20 was prepared wherein Y=NH using a procedure analogous to the procedure described in Example 56 in 35% yield; MS m/e 821 (M+1).

### Example 65

Using a procedure analogous to that described in Example 63 and starting with the product of Example 64, the compound of formula 21 was obtained wherein Y is NH, Y¹ is H, and Y² is 2-methoxybenzyl, in 16% yield; MS m/e 942 (M+1).

### Example 66

Using a procedure analogous to that described in Example 63 and starting with the product of Example 64 and *p*-trifluoromethylbenzaldehyde, the compound of formula 21 was obtained wherein Y is NH, Y¹ is H, and Y² is 4-trifluoromethylbenzyl, in 18% yield; MS m/e 980 (M+1).

### Example 67

A solution of the product from Example 64 (145 mg, 0.18 mmol) and *o*-anisaldehyde (122 mg, 0.9 mmol) in 10 mL of EtOH was stirred overnight at room temperature. EtOH was removed under reduced pressure and the residue was dissolved in 5 mL of MeOH. Sodium borohydride (34 mg, 0.9 mmol) was added and the mixture was stirred at room temperature for 2 hours. MeOH was removed under reduced pressure and the residue was dissolved in CH₂Cl₂ and washed with water and brine. The organic layer was dried over Na₂SO₄ and evaporated. A SiO₂ chromatography (5% MeOH-CH₂Cl₂-0.2% NH₄OH) of the residue gave 104 mg (54%) of the compound of formula 21 wherein Y is NH, and Y¹ and Y² are 2-methoxybenzyl, title compound; MS m/e 1061 (M+1).

### Example 68

Following a procedure analogous to that of Example 61, the compound of formula 20 was obtained where in Y is S, in 63% yield; MS m/e 838 (M+1).

### Example 69

Following a procedure analogous to that of Example 57, the compound of formula 21 was prepared wherein Y is S, Y¹ is H, and Y² is 2-methoxybenzyl, in 28% yield; MS m/e 958 (M=1).

### Example 70

A solution of the product from Example 64 (80 mg, 0.1 mmol) o-anisaldehyde (136 mg, 1 mmol), sodium acetate (64 mg, 0.78 mmol), and sodium triacetoxyborohydride (64 mg, 0.3 mmol) was stirred overnight at room temperature. The resulting solution was diluted with CH₂Cl₂ and washed with a saturated Na₂CO₃ solution and brine. The organic layer was dried over K₂CO₃ and evaporated. The residue was chromatographed on SiO₂ plate (2.5% MeOH-methyl t-butylether-2.5% triethylamine) to give 20 mg (19%) of the compound of formula 21 was prepared wherein Y is S, and Y¹ and Y² are 2-methoxybenzyl, MS m/e 1078 (M+1).

### Example 71

A solution of the product from Example 70 (31 mg, 0.03 mmol) formaldehyde (37% aqueous solution, 83 µL, 1 mmol), and formic acid (18 µL, 0.47 mmol) in 2 mL of CHCl₃ was heated at 61°C for 1 hr. The reaction mixture was diluted with CH₂Cl₂ and wash with a saturated solution of NaHCO₃ and brine. After drying over K₂CO₃, the solvents were removed under reduced pressure. The residue was chromatographed on a SiO₂ plate (5% MeOH-CH₂Cl₂-2.5% triethylamine) to give 14 mg (45%) of the compound of formula 21 wherein Y is S, Y¹ is methyl, and Y² is 2-methoxybenzyl; MS m/e 972 (M+1).

### Example 72

A solution of the compound of formula 12 ( 380 mg, 0.5 mmol) and magnesium perchlorate (223 mg, 1 mmol) in 5mL of MeOH was refluxed under N₂ for 9 days. MeOH was removed under reduced pressure and the residue was dissolved in CH₂Cl₂ and washed with water and brine. The residue was chromatographed on SiO₂ (2.5% MeOH-CH₂Cl₂-0.5% NH₄OH) to give 25 mg (6%) of the configuration indicated below (MS m/e 793 (M+1)):

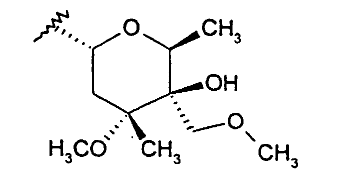

The following scheme illustrates the preparation of compounds referred to in Examples 73-75 below.

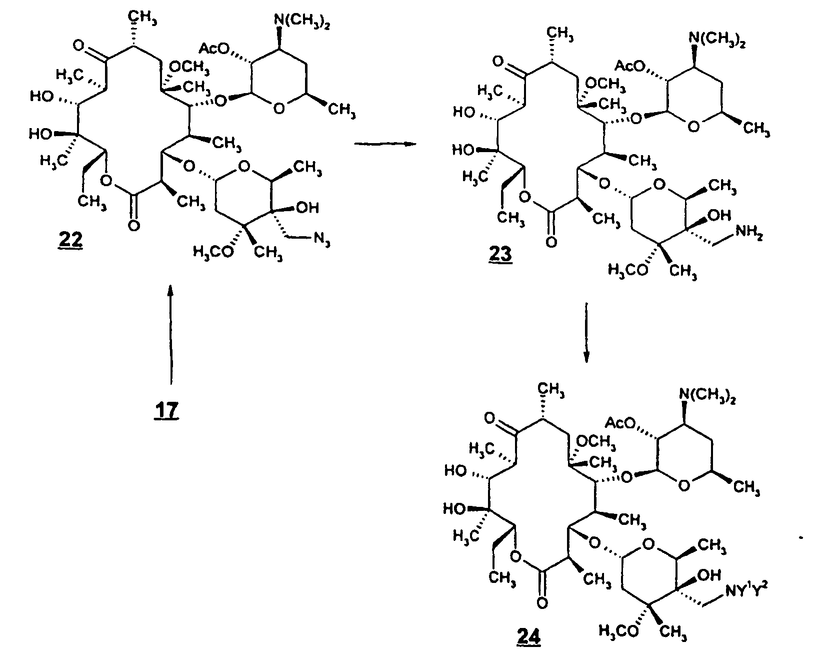

### Example 73

A solution of the compound of formula 17 (500 mg, 0.62 mmol), sodium azide (80 mg, 1.23 mmol), and lithium perchlorate (135 mg, 1.27 mmol) in 5 mL of acetonitrile was refluxed for 4 days. After evaporation of acetonitrile the residue was dissolved in CH₂Cl₂ and washed with water and brine. The CH₂Cl₂ layer was dried over MgSO₄ and concentrated. The residue was dissolved in 5 mL of MeOH and refluxed overnight. The residue obtained after evaporation of the solvent was chromatographed on SiO₂ (4% MeOH-CH₂Cl₂-0.4% NH₄OH) to give 218 mg (44%) of the compound of formula 22; m/e 803 (M+1).

### Example 74

A solution of the compound of formula 23 (250 mg, 0.311 mmol) in 15 mL of EtOH was hydrogenated in the presence of 30 mg 10% Pd/C in a Parr shaker. After 2 hours at room temperature the reaction mixture was filtered through Celite™ and the solvent was removed under reduced pressure. The residue was chromatographed on SiO₂ 98% MeOH-CH₂Cl₂-0.8% NH₄OH) to give 140 mg (58%) of the compound of formula 23; MS m/e 777 (M+1).

### Example 75

Following a procedure analogous to that of Example 57 and using the compound of formula 26 as a starting material, the compound of formula 24 was prepared wherein Y¹ is H and Y² is 2-methoxybenzyl, in 43% yield; MS m/e 897 (M+1).

## Claims

1. A compound of the formula 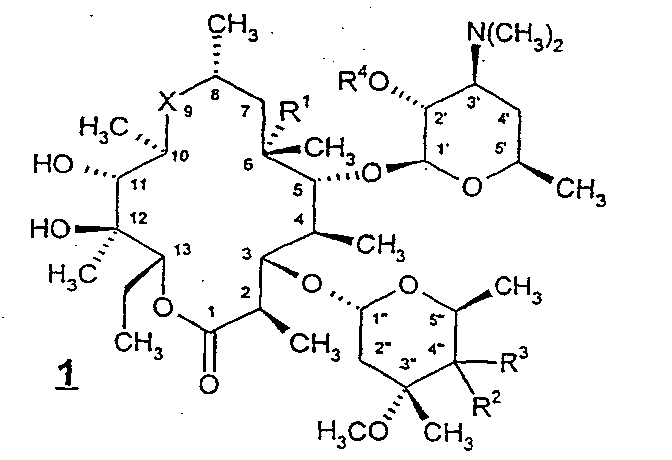 or a pharmaceutically acceptable salt thereof, wherein:
X is -CH(NR⁹R¹⁰)-, -C(O)-, -C(=NOR⁹)-, -CH₂NR⁹-, or -N(C₁-C₆ alkyl)CH₂- wherein the first dash of each of the foregoing X groups is attached to the C-10 carbon of the compound of formula 1 and the last dash of each group is attached to the C-8 carbon of the compound of formula 1;
R¹ is H, hydroxy or methoxy;
R² is hydroxy;
R³ is C₁-C₁₀ alkyl, C₂-C₁₀ alkenyl, C₂-C₁₀ alkynyl, cyano, -CH₂S(O)ₙR⁸ wherein n is an integer ranging from 0 to 2, -CH₂OR⁸, -CH₂N(OR⁹)R⁸, -CH₂NR⁸R¹⁵, -(CH₂)ₘ(C₆-C₁₀ aryl), or -(CH₂)ₘ(5-10 membered heteroaryl), wherein m is an integer ranging from 0 to 4, and wherein the foregoing R³ groups are optionally substituted by 1 to 3 R¹⁶ groups;
or R² and R³ are taken together to form an oxazolyl ring as shown below 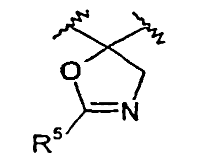 R⁴ is H, -C(O)R⁹, -C(O)OR⁹, -C(O)NR⁹R¹⁰ or a hydroxy protecting group;
R⁵ is -SR⁸, -(CH₂)ₙC(O)R⁸ wherein n is 0 or 1, C₁-C₁₀ alkyl, C₂-C₁₀ alkenyl, C₂-C₁₀ alkynyl, -(CH₂)ₘ(C₆-C₁₀ aryl), or -(CH₂)ₘ(5-10 membered heteroaryl), wherein m is an integer ranging from 0 to 4, and wherein the foregoing R⁵ groups are optionally substituted by 1 to 3 R¹⁶ groups;
each R⁶ and R⁷ is independently H, hydroxy, C₁-C₆ alkoxy, C₁-C₆ alkyl, C₂-C₆ alkenyl, C₂-C₆ alkynyl, -(CH₂)ₘ(C₆-C₁₀ aryl), or -(CH₂)ₘ(5-10 membered heteroaryl), wherein m is an integer ranging from 0 to 4;
each R⁸ is independently H, C₁-C₁₀ alkyl, C₂-C₁₀ alkenyl, C₂-C₁₀ alkynyl, -(CH₂)_{q}CR¹¹R¹²(CH₂)ᵣNR¹³R¹⁴ wherein q and r are each independently an integer ranging from 0 to 3 except q and r are not both 0, -(CH₂)ₘ(C₆-C₁₀ aryl), or -(CH₂)ₘ(5-10 membered heteroaryl), wherein m is an integer ranging from 0 to 4, and wherein the foregoing R⁸ groups, except H, are optionally substituted by 1 to 3 R¹⁶ groups;
or where R⁸ is as -CH₂NR⁸R¹⁵, R¹⁵ and R⁸ may be taken together to form a 4-10 membered monocyclic or polycyclic saturated ring or a 5-10 membered heteroaryl ring, wherein said saturated and heteroaryl rings optionally include 1 or 2 heteroatoms selected from O, S and -N(R⁸)-, in addition to the nitrogen to which R¹⁵ and R⁸ are attached, said saturated ring optionally includes 1 or 2 carbon-carbon double or triple bonds, and said saturated and heteroaryl rings are optionally substituted by 1 to 3 R¹⁶ groups;
each R⁹ and R¹⁰ is independently H or C₁-C₆ alkyl;
each R¹¹, R¹², R¹³ and R¹⁴ is independently selected from H, C₁-C₁₀ alkyl, -(CH₂)ₘ(C₆-C₁₀ aryl), and -(CH₂)ₘ(5-10 membered heteroaryl), wherein m is an integer ranging from 0 to 4, and wherein the foregoing R¹¹, R¹², R¹³ and R¹⁴ groups, except H, are optionally substituted by 1 to 3 R¹⁶ groups;
or R¹¹ and R¹³ are taken together to form -(CH₂)ₚ- wherein p is an integer ranging from 0 to 3 such that a 4-7 membered saturated ring is formed that optionally includes 1 or 2 carbon-carbon double or triple bonds;
or R¹³ and R¹⁴ are taken together to form a 4-10 membered monocyclic or polycyclic saturated ring or a 5-10 membered heteroaryl ring, wherein said saturated and heteroaryl rings optionally include 1 or 2 heteroatoms selected from O, S and -N(R⁸)-, in addition to the nitrogen to which R¹³ and R¹⁴ are attached, said saturated ring optionally includes 1 or 2 carbon-carbon double or triple bonds, and said saturated and heteroaryl rings are optionally substituted by 1 to 3 R¹⁶ groups;
R¹⁵ is H, C₁-C₁₀ alkyl, C₂-C₁₀ alkenyl, or C₂-C₁₀ alkynyl, wherein the foregoing R¹⁵ groups are optionally substituted by 1 to 3 substituents independently selected from halo and -OR⁹;
each R¹⁶ is independently selected from halo, cyano, nitro, trifluoromethyl, azido, -C(O)R¹⁷, -C(O)OR¹⁷, -C(O)OR¹⁷, -OC(O)OR¹⁷, -NR⁶C(O)R⁷, -C(O)NR⁶R⁷, -NR⁶R⁷, hydroxy, C₁-C₆ alkyl, C₁-C₆ alkoxy, -(CH₂)ₘ(C₆-C₁₀ aryl), and -(CH₂)ₘ(5-10 membered heteroaryl), wherein m is an integer ranging from 0 to 4, and wherein said aryl and heteroaryl subsituents are optionally substituted by 1 or 2 substituents independently selected from halo, cyano, nitro, trifluoromethyl, azido, -C(O)R¹⁷, -C(O)OR¹⁷, -C(O)OR¹⁷, -OC(O)OR¹⁷, -NR⁶C(O)R⁷, -C(O)NR⁶R⁷, -NR⁶R⁷, hydroxy, C₁-C₆ alkyl, and C₁-C₆ alkoxy;
each R¹⁷ is independently selected from H, C₁-C₁₀ alkyl, C₂-C₁₀ alkenyl, C₂-C₁₀ alkynyl, -(CH₂)ₘ(C₆-C₁₀ aryl), and -(CH₂)ₘ(5-10 membered heteroaryl), wherein m is an integer ranging from 0 to 4;
with the proviso that R⁸ is not H where R³ is -CH₂S(O)ₙR⁸,

2. The compound of claim 1 wherein R⁴ is H, acetyl, or benzyloxycarbonyl.

3. The compound of claim 2 wherein R¹ is hydroxy, R² is hydroxy, R³ is -CH₂NR¹⁵R⁸ or -CH₂SR⁸.

4. The compound of claim 3 wherein R³ is -CH₂NR¹⁵R⁸ and R¹⁵ and R⁸ are independently selected from H, C₁-C₁₀ alkyl, C₂-C₁₀ alkenyl, and C₂-C₁₀ alkynyl, wherein the foregoing R¹⁵ and R⁸ groups, except H, are optionally substituted by 1 or 2 substituents independently selected from hydroxy, halo and C₁-C₆ alkoxy.

5. The compound of claim 4 wherein R¹⁵ and R⁸ are each independently selected from H, methyl, ethyl, allyl, n-butyl, isobutyl, 2-methoxyethyl, cyclopentyl, 3-methoxypropyl, 3-ethoxypropyl, n-propyl, isopropyl, 2-hydroxyethyl, cyclopropyl, 2,2,2-trifluoroethyl, 2-propynyl, *sec*-butyl, *tert*-butyl, and n-hexyl.

6. The compound of claim 2 wherein R¹ is hydroxy, R² is hydroxy, R³ is -CH₂NHR⁸, and R⁸ is -(CH₂)ₘ(C₆-C₁₀ aryl) wherein m is an integer ranging from 0 to 4.

7. The compound of claim 6 wherein R⁸ is phenyl or benzyl.

8. The compound of claim 2 wherein R¹ is hydroxy, R² is hydroxy, R³ is -CH₂N¹⁵R⁸, and R¹⁵ and R⁸ are taken together to form a 4-7 membered saturated ring.

9. The compound of claim 8 wherein R¹⁵ and R⁸ are taken together to form a piperidino, trimethyleneimino, or morpholino ring.

10. The compound of claim 2 wherein R¹ is hydroxy, R² is hydroxy, R³ is -CH₂NR¹⁵R⁸, and R¹⁵ and R⁸ are taken together to form a 5-10 membered heteroaryl ring optionally substituted by 1 or 2 C₁-C₆ alkyl groups.

11. The compound of claim 10 wherein R¹⁵ and R⁸ are taken together to form a pyrrolidino, triazolyl, or imidazolyl ring wherein said heteroaryl groups are optionally substituted by 1 or 2 methyl groups.

12. The compound of claim 2 wherein R¹ is hydroxy, R² is hydroxy, R³ is -CH₂SR⁸, and R⁸ is selected from C₁-C₁₀ alkyl, C₂-C₁₀ alkenyl, and C₂-C₁₀ alkynyl, wherein said R⁸ groups are optionally substituted by 1 or 2 substituents independently selected from hydroxy, halo and C₁-C₆ alkoxy.

13. The compound of claim 12 wherein R⁸ is methyl, ethyl, or 2-hydroxyethyl.

14. The compound of claim 2 wherein R¹ is hydroxy, R² is hydroxy, and R³ is selected from C₁-C₁₀ alkyl, C₂-C₁₀ alkenyl, and C₂-C₁₀ alkynyl, wherein said R³ groups are optionally substituted by 1 or 2 subsbtuents independently selected from hydroxy, -C(O)R¹⁷, -NR⁶R⁷, halo, cyano, azido, 5-10 membered heteroaryl, and C₁-C₆ alkoxy.

15. The compound of claim 14 wherein R³ is methyl, allyl, vinyl, ethynyl, 1-methyl-1-propenyl, 3-methoxy-1-propynyl, 3-dimethylamino-1-propynyl, 2-pyridylethynyl, 1-propynyl, 3-hydroxy-1-propynyl, 3-hydroxy-1-propenyl, 3-hydroxypropyl, 3-methoxy-1-propenyl, 3-methoxypropyl, 1-propynyl, n-butyl, ethyl, propyl, 2-hydroxyethyl, azidomethyl, formylmethyl, 6-cyano-1-pentynyl, 3-dimethylamino-1-propenyl, or 3-dimethylaminopropyl.

16. The compound of claim 2 wherein R¹ is hydroxy, R² is hydroxy, and R³ is -(CH₂)ₘ(5-10 membered heteroaryl) wherein m is an integer ranging from 0 to 4.

17. The compound of claim 16 wherein R³ is 2-thienyl, 2-pyridyl, 1-methyl-2-imidazolyl, 2-furyl, or 1-methyl-2-pyrrolyl.

18. The compound of claim 2 wherein R¹ is hydroxy, R² is hydroxy, and R³ is -(CH₂)ₘ(C₆-C₁₀ aryl) wherein m is an integer ranging from 0 to 4.

19. The compound of claim 18 wherein R³ is phenyl.

20. The compound of claim 2 wherein R² and R³ are taken together to form an oxazolyl ring as shown below

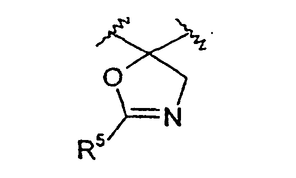

21. The compound of claim 2 wherein R³ is selected from the following: 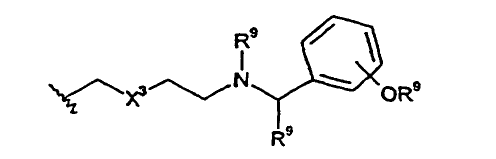 wherein X³ is O, S or -N(R¹⁵)-, R⁹ and R¹⁵ are as defined in claim 1, and the -OR⁹ group may be attached at any available carbon on the phenyl group.

22. A compound as claimed in claim 1 for use as a medicament.

23. The use of a compound as claimed in claim 1 for the manufacture of a medicament for the treatment of a bacterial or protozoal infection in a mammal, fish or bird

24. A composition comprising a compound as claimed in claim 1 and a pharmaceutically acceptable carrier.

25. A composition as claimed in claim 24, wherein said composition is for the treatment of a bacterial or protozoal infection in a mammal, fish or bird.

26. A method of preparing a compound of the formula 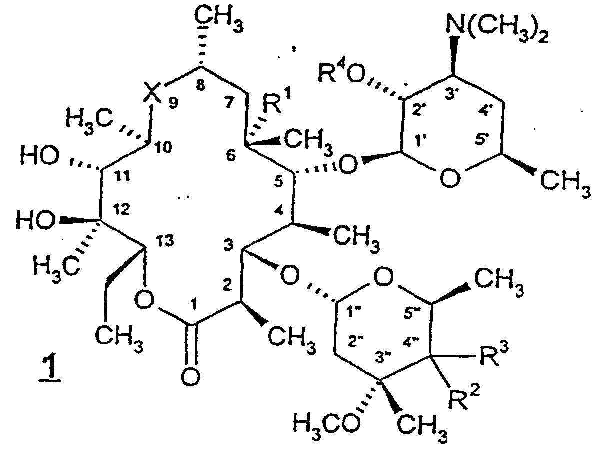 or a pharmaceutically acceptable salt thereof, wherein:
X is -CH(NR⁹R¹⁰)-, -C(O)-, -C(=NOR⁹)-, -CH₂NR⁹-, or -N(C₁-C₆ alkyl)CH₂- wherein the first dash of each of the foregoing X groups is attached to the C-10 carbon of the compound of formula 1 and the last dash of each group is attached to the C-8 carbon of the compound of formula 1;
R¹ is H, hydroxy or methoxy;
R² is hydroxy;
R³ is C₁-C₁₀ alkyl, C₂-C₁₀ alkenyl, C₂-C₁₀ alkynyl, cyano, -CH₂S(O)ₙR⁸ wherein n is an
integer ranging from 0 to 2, -CH₂OR⁸, -CH₂N(OR⁹)R⁸, -CH₂NR⁸R¹⁵, -(CH₂)ₘ(C₆-C₁₀ aryl), or -(CH₂)ₘ(5-10 membered heteroaryl), wherein m is an integer ranging from 0 to 4, and wherein the foregoing R³ groups are optionally substituted by 1 to 3 R¹⁶ groups;
or R² and R³ are taken together to form an oxazolyl ring as shown below 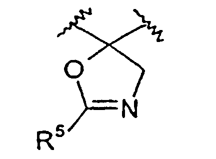 R⁴ is H, -C(O)R⁹, -C(O)OR⁹, -C(O)NR⁹R¹⁰ or a hydroxy protecting group;
R⁵ is -SR⁸, -(CH₂)ₙC(O)R⁸ wherein n is 0 or 1, C₁-C₁₀ alkyl, C₂-C₁₀ alkenyl, C₂-C₁₀ alkynyl, -(CH₂)ₘ(C₆-C₁₀ aryl), or -(CH₂)ₘ(5-10 membered heteroaryl), wherein m is an integer ranging from 0 to 4, and wherein the foregoing R⁵ groups are optionally substituted by 1 to 3 R¹⁶ groups;
each R⁶ and R⁷ is independently H, hydroxy, C₁-C₆ alkoxy, C₁-C₆ alkyl, C₂-C₆ alkenyl, C₂-C₆ alkynyl, -(CH₂)ₘ(C₆-C₁₀ aryl), or -(CH₂)ₘ(5-10 membered heteroaryl), wherein m is an integer ranging from 0 to 4;
each R⁸ is independently H, C₁-C₁₀ alkyl; C₂-C₁₀ alkenyl, C₂-C₁₀ alkynyl, -(CH₂)_{q}CR¹¹R¹²(CH₂)ᵣNR¹³R¹⁴ wherein q and r are each independently an integer ranging from 0 to 3 except q and r are not both 0, -(CH₂)ₘ(C₆-C₁₀ aryl), or -(CH₂)ₘ(5-10 membered heteroaryl), wherein m is an integer ranging from 0 to 4, and wherein the foregoing R⁸ groups, except H, are optionally substituted by 1 to 3 R¹⁶ groups;
or where R⁸ is as -CH₂NR⁸R¹⁵, R⁵ and R⁸ may be taken together to form a 4-10 membered monocyclic or polycyclic saturated ring or a 5-10 membered heteroaryl ring, wherein said saturated and heteroaryl rings optionally include 1 or 2 heteroatoms selected from O, S and -N(R⁸)-, in addition to the nitrogen to which R¹⁵ and R⁸ are attached, said saturated ring optionally includes 1 or 2 carbon-carbon double or triple bonds, and said saturated and heteroaryl rings are optionally substituted by 1 to 3 R¹⁶ groups;
each R⁹ and R¹⁰ is independently H or C₁-C₆ alkyl;
each R¹¹, R¹², R¹³ and R¹⁴ is independently selected from H, C₁-C₁₀ alkyl, -(CH₂)ₘ(C₆-C₁₀ aryl), and -(CH₂)ₘ(5-10 membered heteroaryl), wherein m is an integer ranging from 0 to 4, and wherein the foregoing R¹¹, R¹², R¹³ and R¹⁴ groups, except H, are optionally substituted by 1 to 3 R¹⁶ groups;
or R¹¹ and R¹³ are taken together to form -(CH₂)ₚ- wherein p is an integer ranging from 0 to 3 such that a 4-7 membered saturated ring is formed that optionally includes 1 or 2 carbon-carbon double or triple bonds;
or R¹³ and R¹⁴ are taken together to form a 4-10 membered monocyclic or polycyclic saturated ring or a 5-10 membered heteroaryl ring, wherein said saturated and heteroaryl rings optionally include 1 or 2 heteroatoms selected from O, S and -N(R⁸)-, in addition to the nitrogen to which R¹³ and R¹⁴ are attached, said saturated ring optionally includes 1 or 2 carbon-carbon double or triple bonds, and said saturated and heteroaryl rings are optionally substituted by 1 to 3 R¹⁶ groups;
R¹⁵ is H, C₁-C₁₀ alkyl, C₂-C₁₀ alkenyl, or C₂-C₁₀ alkynyl, wherein the foregoing R¹⁵ groups are optionally substituted by 1 to 3 substituents independently selected from halo and -OR⁹;
each R¹⁶ is independently selected from halo, cyano, nitro, trifluoromethyl, azido, -C(O)R¹⁷, -C(O)OR¹⁷, -C(O)OR¹⁷, -OC(O)OR¹⁷, -NR⁶C(O)R⁷, -C(O)NR⁶R⁷, -NR⁶R⁷, hydroxy, C₁-C₆ alkyl, C₁-C₆ alkoxy, -(CH₂)ₘ(C₆-C₁₀ aryl), and -(CH₂)ₘ(5-10 membered heteroaryl), wherein m is an integer ranging from 0 to 4, and wherein said aryl and heteroaryl subsituents are optionally substituted by 1 or 2 substituents independently selected from halo, cyano, nitro, trifluoromethyl, azido, -C(O)R¹⁷, -C(O)OR¹⁷, -C(O)OR¹⁷, -OC(O)OR¹⁷, -NR⁶C(O)R⁷, -C(O)NR⁶R⁷, -NR⁶R⁷, hydroxy, C₁-C₆ alkyl, and C₁-C₆ alkoxy;
each R¹⁷ is independently selected from H, C₁-C₁₀ alkyl, C₂-C₁₀ alkenyl, C₂-C₁₀ alkynyl, -(CH₂)ₘ(C₆-C₁₀ aryl), and -(CH₂)ₘ(5-10 membered heteroaryl), wherein m is an integer ranging from 0 to 4;
with the proviso that R⁸ is not H where R³ is -CH₂S(O)ₙR⁸;
which comprises treating a compound of the formula 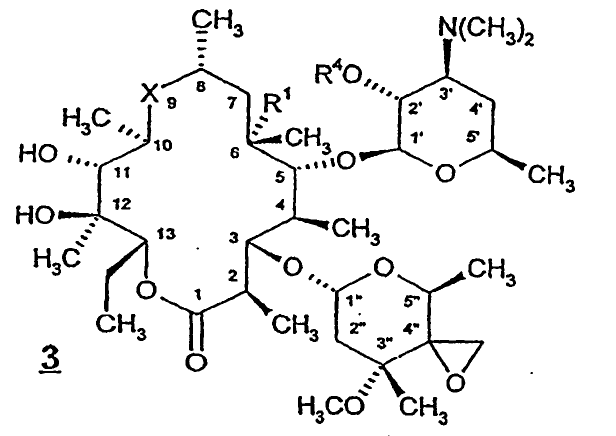 wherein X, R¹ and R⁴ are as defined above, with a compound of the formula HOR⁸, HSR⁸ or HNR¹⁵R⁸, wherein n, R¹⁵ and R⁸ are as defined above, wherein if said compound of formula HSR⁸ is used the resulting R³ group of formula -CH₂SR⁸ is optionally oxidised to -CH₂(O)R⁸ or -CH₂S(O)₂R⁸.

27. The method of claim 26 wherein the compound of formula 3 is prepared by treating a compound of the formula 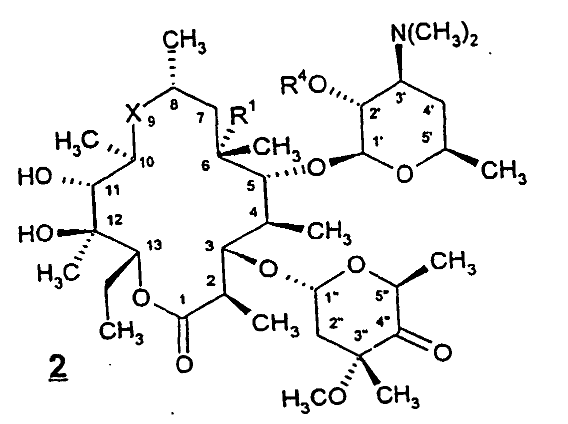 wherein X, R¹ and R⁴ are as defined in claim 24, with (CH₃)₃S(O)ₙX², wherein n is 0 or 1 and X² is halo, -BF₄ or -PF₆, in the presence of a base.

28. The method of claim 27 to produce the compound of claim 1 wherein X² is iodo or BF₄ and said base is selected from potassium tert-butoxide, sodium tert-butoxide, sodium ethoxide, sodium hydride, 1,1,3,3-tetramethylguanidine, 1,8-diazabicyclo[5.4.0]undec-7-ene, 1,5-diazabicylo[4.3.0]non-5-ene, potassium hexamethyldisilazide (KHMDS), potassium ethoxide, and sodium methoxide.

29. A compound of the formula 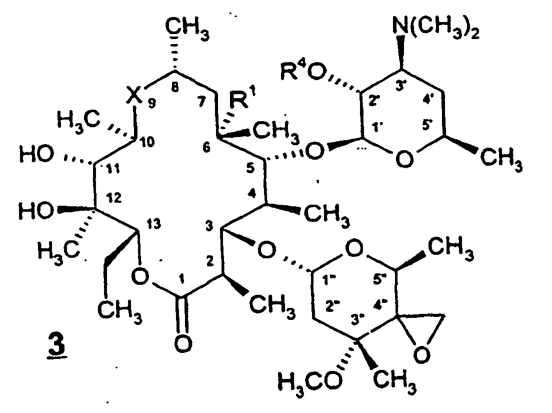 or a pharmaceutically acceptable salt thereof, wherein
X is -CH(NR⁹R¹⁰)-, -C(O)-, -C(=NOR⁹), -CH₂NR⁹-, or -N(C₁-C₆ alkyl)CH₂- wherein the first dash of each for the foregoing X groups is attached to the C-10 carbon of the compound of formula 3 and the last dash of each group is attached to the C-8 carbon of the of the compound of formula 3;
R¹ is H, hydroxy or methoxy:
R⁴ is H, -C(O)R⁹, -C(O)OR⁹, -C(O)NR⁹R¹⁰ or a hydroxy protecting group;
each R⁹ and R¹⁰ is independently H or C₁-C₆ alkyl.

## Patentansprüche

1. Verbindung der Formel 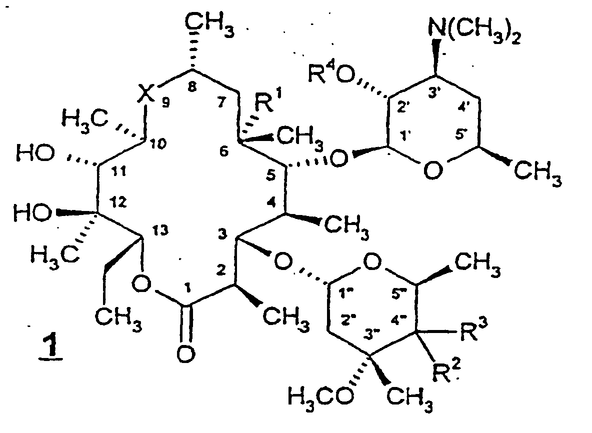 oder ein pharmazeutisch akzeptables Salz derselben, worin:
X -CH(NR⁹R¹⁰), -C(O)- , -C(=NOR⁹), -CH₂NR⁹- oder -N(C₁-C₆-Alkyl)CH₂- bedeutet, wobei der erste Strich von jeder der im vorhergehenden genannten X-Gruppen an den C-10-Kohlenstoff der Verbindung der Formel 1 gebunden ist und der letzte Strich jeder Gruppe an den C-8-Kohlenstoff der Verbindung der Formel 1 gebunden ist; R¹ H, Hydroxy oder Methoxy bedeutet;
R² Hydroxy bedeutet;
R³ C₁-C₁₀-Alkyl, C₂-C₁₀-Alkenyl, C₂-C₁₀-Alkinyl, Cyano, -CH₂S(O)ₙR⁸, wobei n eine ganze Zahl im Bereich von 0 bis 2 ist, -CH₂OR⁸, -CH₂N(OR⁹)R⁸, -CH₂NR⁸R¹⁵, -(CH₂)ₘ(C₆-C₁₀-Aryl) oder -(CH₂)ₘ(5-10-gliedriges Heteroaryl), wobei m eine ganze Zahl im Bereich von 0 bis 4 ist, bedeutet und wobei die im vorhergehenden genannten R³-Gruppen optional mit 1 bis 3 R¹⁶-Gruppen substituiert sind;
oder R² und R³ zusammengenommen einen Oxazolylring bilden, der im folgenden angegeben ist: 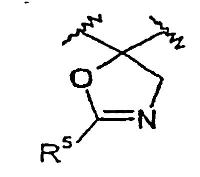
R⁴ H, -C(O)R⁹, -C(O)OR⁹, -C(O)NR⁹R¹⁰ oder eine Hydroxyschutzgruppe bedeutet;
R⁵ -SR⁸, -(CH₂)ₙC(O)R⁸, wobei n 0 oder 1 ist, C₁-C₁₀-Alkyl, C₂-C₁₀-Alkenyl , C₂-C₁₀-Alkinyl, -(CH₂)ₘ(C₆-C₁₀-Aryl) oder -(CH₂)ₘ(5-10-gliedriges Heteroaryl), wobei m eine ganze Zahl im Bereich von 0 bis 4 ist, bedeutet und wobei die im vorhergehenden genannten R⁵-Gruppen optional mit 1 bis 3 R¹⁶-Gruppen substituiert sind; jedes R⁶ und R⁷ unabhängig voneinander H, Hydroxy, C₁-C₆-Alkoxy, C₁-C₆-Alkyl , C₂-C₆-Alkenyl, C₂-C₆-Alkinyl, -(CH₂)ₘ(C₆-C₁₀-Aryl) oder -(CH₂)ₘ(5-10-gliedriges Heteroaryl), wobei m eine ganze Zahl im Bereich von 0 bis 4 ist, bedeutet;
jedes R⁸ unabhängig voneinander H, C₁-C₁₀-Alkyl, C₂-C₁₀-Alkenyl, C₂-C₁₀-Alkinyl , -(CH₂)_{q}CR¹¹R¹²(CH₂)ᵣNR¹³R¹⁴, wobei q und r jeweils unabhängig voneinander eine ganze Zahl im Bereich von 0 bis 3 sind, mit Ausnahme davon, dass q und r nicht beide 0 sind, -(CH₂)ₘ(C₆-C₁₀-Aryl) oder -(CH₂)ₘ(5-10-gliedriges Heteroaryl), wobei m eine ganze Zahl im Bereich von 0 bis 4 ist, bedeutet und wobei die im vorhergehenden genannten R⁸-Gruppen mit Ausnahme von H optional mit 1 bis 3 R¹⁶-Gruppen substituiert sind;
oder, wenn R⁸ -CH₂NR⁸R¹⁵ ist, R¹⁵ und R⁸ zusammengenommen einen 4-10-gliedrigen monocyclischen oder polycyclischen gesättigten Ring oder einen 5-10-gliedrigen Heteroarylring bilden können, wobei die gesättigten und Heteroarylringe optional 1 oder 2 Heteroatome, die aus O, S und -N(R⁸)- ausgewählt sind, zusätzlich zu dem Stickstoff, an den R¹⁵ und R⁸ gebunden sind, umfassen, der gesättigte Ring optional 1 oder 2 Kohlenstoff-Kohlenstoff-Doppel- oder -Dreifachbindungen umfasst und die gesättigten und Heteroarylringe optional mit 1 bis 3 R¹⁶-Gruppen substituiert sind; jedes R⁹ und R¹⁰ unabhängig voneinander H oder C₁-C₆-Alkyl bedeutet;
jedes R¹¹, R¹², R¹³ und R¹⁴ unabhängig voneinander aus H, C₁-C₁₀-Alkyl, -(CH₂)ₘ(C₆-C₁₀-Aryl) und -(CH₂)ₘ(5-10-gliedriges Heteroaryl), wobei m eine ganze Zahl im Bereich von 0 bis 4 ist, ausgewählt ist und wobei die im vorhergehenden genannten R¹¹- R¹²-, R¹³- und R¹⁴-Gruppen mit Ausnahme von H optional mit 1 bis 3 R¹⁶-Gruppen substituiert sind;
oder R¹¹ und R¹² zusammengenommen -(CH₂)ₚ-, wobei p eine ganze Zahl im Bereich von 0 bis 3 ist, derart bilden, dass ein 4-7-gliedriger gesättigter Ring gebildet wird, der optional 1 oder 2 Kohlenstoff-Kohlenstoff-Doppel- oder -Dreifachbindungen umfasst;
oder R¹³ und R¹⁴ zusammengenommen einen 4-10-gliedrigen monocyclischen oder polycyclischen gesättigten Ring oder einen 5-10-gliedrigen Heteroarylring bilden, wobei die gesättigten und Heteroarylringe optional 1 oder 2 Heteroatome, die aus O, S und -N(R⁸)- ausgewählt sind, zusätzlich zu dem Stickstoff, an den R¹³ und R¹⁴ gebunden sind, umfassen, wobei der gesättigte Ring optional 1 oder 2 Kohlenstoff-Kohlenstoff-Doppel- oder -Dreifachbindungen umfasst und die gesättigten und Heteroarylringe optional mit 1 bis 3 R¹⁶-Gruppen substituiert sind;
R¹⁵ H, C₁-C₁₀-Alkyl, C₂-C₁₀-Alkenyl oder C₂-C₁₀-Alkinyl bedeutet, wobei die im vorhergehenden genannten R¹⁵-Gruppen optional mit 1 bis 3 Substituenten substituiert sind, die unabhängig voneinander aus Halogen und -OR⁹ ausgewählt sind;
jedes R¹⁶ unabhängig voneinander aus Halogen, Cyano, Nitro, Trifluormethyl, Azido, -C(O)R¹⁷, -C(O)OR¹⁷, -C(O)OR¹⁷, -OC(O)OR¹⁷, -NR⁶C(O)R⁷, -C(O)NR⁶R⁷, -NR⁶R⁷, Hydroxy, C₁-C₆-Alkyl, C₁-C₆-Alkoxy, -(CH₂)ₘ(C₆-C₁₀-Aryl) und -(CH₂)ₘ(5-10-gliedriges Heteroaryl), wobei m eine ganze Zahl im Bereich von 0 bis 4 ist, ausgewählt ist, und wobei die Aryl- und Heteroarylsubstituenten optional mit 1 oder 2 Substituenten substituiert sind, die unabhängig voneinander aus Halogen, Cyano, Nitro, Trifluormethyl, Azido, -C(O)R¹⁷, -C(O)OR¹⁷, -C(O)OR¹⁷, -OC(O)OR¹⁷, -NR⁶C(O)R⁷, -C(O)NR⁶R⁷, -NR⁶R⁷, Hydroxy, C₁-C₆-Alkyl und C₁-C₆-Alkoxy ausgewählt sind; jedes R¹⁷ unabhängig voneinander aus H, C₁-C₁₀-Alkyl, C₂-C₁₀-Alkenyl , C₂-C₁₀-Alkinyl , -(CH₂)ₘ(C₆-C₁₀-Aryl) und -(CH₂)ₘ(5-10-gliedriges Heteroaryl), wobei m eine ganze Zahl im Bereich von 0 bis 4 ist, ausgewählt ist;
mit dem Vorbehalt, dass R⁸ nicht H ist, wenn R³ -CH₂S(O)ₙR⁸ ist.

2. Verbindung nach Anspruch 1, wobei R⁴ H, Acetyl oder Benzyloxycarbonyl ist.

3. Verbindung nach Anspruch 2, wobei R¹ Hydroxy ist, R² Hydroxy ist, R³ -CH₂NR¹⁵R⁸ oder -CH₂SR⁸ ist.

4. Verbindung nach Anspruch 3, wobei R³ CH₂NR¹⁵R⁸ ist und R¹⁵ und R⁸ unabhängig voneinander aus H, C₁-C₁₀-Alkyl, C₂-C₁₀-Alkenyl und C₂-C₁₀-Alkinyl ausgewählt sind, wobei die im vorhergehenden genannten R¹⁵- und R⁸-Gruppen mit Ausnahme von H optional mit 1 oder 2 Substituenten substituiert sind, die unabhängig voneinander aus Hydroxy, Halogen und C₁₇C₆-Alkoxy ausgewählt sind.

5. Verbindung nach Anspruch 4, wobei R¹⁵ und R⁸ jeweils unabhängig voneinander aus H, Methyl, Ethyl, Allyl, n-Butyl, Isobutyl, 2-Methoxyethyl, Cyclopentyl, 3-Methoxypropyl, 3-Ethoxypropyl, n-Propyl, Isopropyl, 2-Hydroxyethyl, Cyclopropyl, 2,2,2-Trifluorethyl, 2-Propinyl, sek-Butyl, tert-Butyl und n-Hexyl ausgewählt sind.

6. Verbindung nach Anspruch 2, wobei R¹ Hydroxy ist, R² Hydroxy ist, R³ -CH₂NHR⁸ ist und R⁸-(CH₂)ₘ(C₆-C₁₀-Aryl), wobei m eine ganze Zahl im Bereich von 0 bis 4 ist, ist.

7. Verbindung nach Anspruch 6, wobei R⁸ Phenyl oder Benzyl ist.

8. Verbindung nach Anspruch 2, wobei R¹ Hydroxy ist, R² Hydroxy ist, R³ -CH₂NR¹⁵R⁸ ist und R¹⁵ und R⁸ zusammengenommen einen 4-7-gliedrigen gesättigten Ring bilden.

9. Verbindung nach Anspruch 8, wobei R¹⁵ und R⁸ zusammengenommen einen Piperidino-, Trimethylenimino- oder Morpholinoring bilden.

10. Verbindung nach Anspruch 2, wobei R¹ Hydroxy ist, R² Hydroxy ist, R³ -CH₂NR¹⁵R⁸ ist und R¹⁵ und R⁸ zusammengenommen einen 5-10-gliedrigen Heteroarylring, der optional mit 1 oder 2 C₁-C₆-Alkylgruppen substituiert ist, bilden.

11. Verbindung nach Anspruch 10, wobei R¹⁵ und R⁸ zusammengenommen einen Pyrrolidino-, Triazolyl- oder Imidazolylring bilden, wobei die Heteroarylgruppen optional mit 1 oder 2 Methylgruppen substituiert sind.

12. Verbindung nach Anspruch 2, wobei R¹ Hydroxy ist, R² Hydroxy ist, R³ -CH₂SR⁸ ist und R³ aus C₁-C₁₀-Alkyl, C₂-C₁₀-Alkenyl und C₂-C₁₀-Alkinyl ausgewählt ist, wobei die R⁸-Gruppen optional mit 1 oder 2 Substituenten substituiert sind, die unabhängig voneinander aus Hydroxy, Halogen und C₁-C₆-Alkoxy ausgewählt sind.

13. Verbindung nach Anspruch 12, wobei R⁸ Methyl, Ethyl oder 2-Hydroxyethyl ist.

14. Verbindung nach Anspruch 2, wobei R¹ Hydroxy ist, R² Hydroxy ist, R³ aus C₁-C₁₀-Alkyl, C₂-C₁₀-Alkenyl und C₂-C₁₀-Alkinyl ausgewählt ist, wobei die R³-Gruppen optional mit 1 oder 2 Substituenten substituiert sind, die unabhängig voneinander aus Hydroxy, -C(O)R¹⁷, -NR⁶R⁷, Halogen, Cyano, Azido, 5-10-gliedrigem Heteroaryl und C₁-C₆-Alkoxy ausgewählt sind.

15. Verbindung nach Anspruch 14, wobei R³ Methyl, Allyl, Vinyl, Ethinyl, 1-Methyl-1-propenyl, 3-Methoxy-1-propinyl, 3-Dimethylamino-1-propinyl, 2-Pyridylethinyl, 1-Propinyl, 3-Hydroxy-1-propinyl, 3-Hydroxy-1-propenyl, 3-Hydroxypropyl, 3-Methoxy-1-propenyl, 3-Methoxypropyl, 1-Propinyl, n-Butyl, Ethyl, Propyl, 2-Hdroxyethyl, Azidomethyl, Formylmethyl, 6-Cyano-1-pentinyl, 3-Dimethylamino-1-propenyl oder 3-Dimethylaminopropyl ist.

16. Verbindung nach Anspruch 2, wobei R¹ Hydroxy ist, R² Hydroxy ist und R³ -(CH₂)ₘ(5-10-gliedriges Heteroaryl), wobei m eine ganze Zahl im Bereich von 0 bis 4 ist, ist.

17. Verbindung nach Anspruch 16, wobei R³ 2-Thienyl, 2-Pyridyl, 1-Methyl-2-imidazolyl, 2-Furyl oder 1-Methyl-2-pyrrolyl ist.

18. Verbindung nach Anspruch 2, wobei R¹ Hydroxy ist, R² Hydroxy ist und R³ -(CH₂)ₘ(C₆-C₁₀-Aryl), wobei m eine ganze Zahl im bereich von 0 bis 4 ist, ist.

19. Verbindung nach Anspruch 18, wobei R³ Phenyl ist.

20. Verbindung nach Anspruch 2, wobei R² und R³ zusammengenommen einen Oxazolylring bilden, der im folgenden angegeben ist:

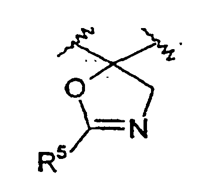

21. Verbindung nach Anspruch 2, wobei R³ aus dem folgenden ausgewählt ist: 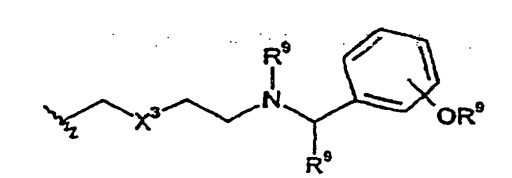 worin X³ O, S oder -N(R¹⁵)- ist, R⁹ und R¹⁵ wie in Anspruch 1 definiert sind und die -OR⁹-Gruppe an einen verfügbaren Kohlenstoff an der Phenylgruppe gebunden sein kann.

22. Verbindung gemäß Anspruch 1 zur Verwendung als Medikament.

23. Verwendung einer Verbindung gemäß Anspruch 1 zur Herstellung eines Medikaments zur Behandlung einer Bakterien- oder Protozoeninfektion bei einem Säuger, Fisch oder Vogel.

24. Zusammensetzung, die eine Verbindung gemäß Anspruch 1 und einen pharmazeutisch akzeptablen Träger umfasst.

25. Zusammensetzung gemäß Anspruch 24, wobei die Zusammensetzung zur Behandlung einer Bakterien- oder Protozoeninfektion bei einem Säuger, Fisch oder Vogel dient.

26. Verfahren zur Herstellung einer Verbindung der Formel 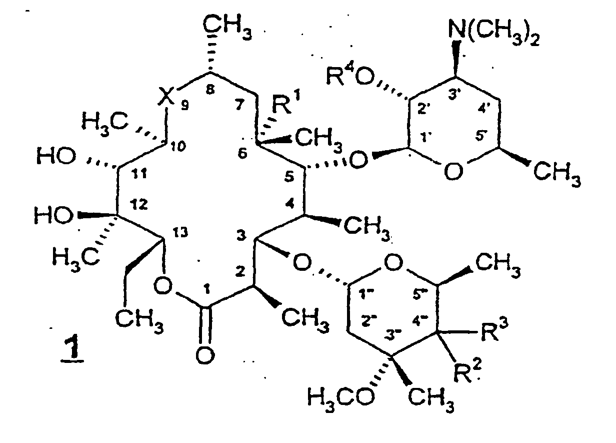 oder eines pharmazeutisch akzeptablen Salzes derselben, worin:
X -CH(NR⁹R¹⁰), -C(O)-, -C(=NOR⁹)-, -CH₂NR⁹- oder -N(C₁-C₆-Alkyl)CH₂- bedeutet, wobei der erste Strich von jeder der im vorhergehenden genannten X-Gruppen an den C-10-Kohlenstoff der Verbindung der Formel 1 gebunden ist und der letzte Strich jeder Gruppe an den C-8-Kohlenstoff der Verbindung der Formel 1 gebunden ist;
R¹ H, Hydroxy oder Methoxy bedeutet;
R² Hydroxy bedeutet;
R³ C₁-C₁₀-Alkyl, C₂-C₁₀-Alkenyl, C₂-C₁₀-Alkinyl, Cyano, -CH₂S(O)ₙR⁸, wobei n eine ganze Zahl im Bereich von 0 bis 2 ist, -CH₂OR⁸, -CH₂N(OR⁹)R⁸, -CH₂NR⁸R¹⁵, -(CH₂)ₘ(C₆-C₁₀-Aryl) oder -(CH₂)ₘ(5-10-gliedriges Heteroaryl), wobei m eine ganze Zahl im Bereich von 0 bis 4 ist, bedeutet und wobei die im vorhergehenden genannten R³-Gruppen optional mit 1 bis 3 R¹⁶-Gruppen substituiert sind;
oder R² und R³ zusammengenommen einen Oxazolylring bilden, der im folgenden angegeben ist: 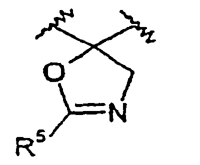
R⁴ H, -C(O)R⁹, -C(O)OR⁹, -C(O)NR⁹R¹⁰ oder eine Hydroxyschutzgruppe bedeutet;
R⁵ -SR⁸, -(CH₂)ₙC(O)R⁸, wobei n 0 oder 1 ist, C₁-C₁₀-Alkyl, C₂-C₁₀-Alkenyl, C₂-C₁₀-Alkinyl, -(CH₂)ₘ(C₆-C₁₀-Aryl) oder -(CH₂)ₘ(5-10-gliedriges Heteroaryl), wobei m eine ganze Zahl im Bereich von 0 bis 4 ist, bedeutet und wobei die im vorhergehenden genannten R⁵-Gruppen optional mit 1 bis 3 R¹⁶-Gruppen substituiert sind; jedes R⁶ und R⁷ unabhängig voneinander H, Hydroxy, C₁-C₆-Alkoxy, C₁-C₆-Alkyl, C₂-C₆-Alkenyl, C₂-C₆-Alkinyl, -(CH₂)ₘ(C₆-C₁₀-Aryl) oder -(CH₂)ₘ(5-10-gliedriges
Heteroaryl), wobei m eine ganze Zahl im Bereich von 0 bis 4 ist, bedeutet;
jedes R⁸ unabhängig voneinander H, C₁-C₁₀-Alkyl, C₂-C₁₀-Alkenyl, C₂-C₁₀-Alkinyl, -(CH₂)_{q}CR¹¹R¹²(CH₂)ᵣNR¹³R¹⁴, wobei q und r jeweils unabhängig voneinander eine ganze Zahl im Bereich von 0 bis 3 sind, mit Ausnahme davon, dass q und r nicht beide 0 sind, -(CH₂)ₘ(C₆-C₁₀-Aryl) oder -(CH₂)ₘ(5-10-gliedriges Heteroaryl), wobei m eine ganze Zahl im Bereich von 0 bis 4 ist, bedeutet und wobei die im vorhergehenden genannten R⁸-Gruppen mit Ausnahme von H optional mit 1 bis 3 R¹⁶-Gruppen substituiert sind;
oder, wenn R⁸ -CH₂NR⁸R¹⁵ ist, R¹⁵ und R⁸ zusammengenommen einen 4-10-gliedrigen monocyclischen oder polycyclischen gesättigten Ring oder einen 5-10-gliedrigen Heteroarylring bilden können, wobei die gesättigten und Heteroarylringe optional 1 oder 2 Heteroatome, die aus O, S und -N(R⁸)- ausgewählt sind, zusätzlich zu dem Stickstoff, an den R¹⁵ und R⁸ gebunden sind, umfassen, der gesättigte Ring optional 1 oder 2 Kohlenstoff-Kohlenstoff-Doppel- oder -Dreifachbindungen umfasst und die gesättigten und Heteroarylringe optional mit 1 bis 3 R¹⁶-Gruppen substituiert sind;
jedes R⁹ und R¹⁰ unabhängig voneinander H oder C₁-C₆-Alkyl bedeutet;
jedes R¹¹, R¹², R¹³ und R¹⁴ unabhängig voneinander aus H, C₁-C₁₀-Alkyl, -(CH₂)ₘ(C₆-C₁₀-Aryl) und -(CH₂)ₘ(5-10-gliedriges Heteroaryl), wobei m eine ganze Zahl im Bereich von 0 bis 4 ist, ausgewählt ist und wobei die im vorhergehenden genannten R¹¹-, R¹²-, R¹³- und R¹⁴-Gruppen mit Ausnahme von H optional mit 1 bis 3 R¹⁶-Gruppen substituiert sind;
oder R¹¹ und R¹² zusammengenommen -(CH₂)ₚ-, wobei p eine ganze Zahl im Bereich von 0 bis 3 ist, derart bilden, dass ein 4-7-gliedriger gesättigter Ring gebildet wird, der optional 1 oder 2 Kohlenstoff-Kohlenstoff-Doppel- oder -Dreifachbindungen umfasst;
oder R¹³ und R¹⁴ zusammengenommen einen 4-10-gliedrigen monocyclischen oder polycyclischen gesättigten Ring oder einen 5-10-gliedrigen Heteroarylring bilden, wobei die gesättigten und Heteroarylringe optional 1 oder 2 Heteroatome, die aus O, S und -N(R⁸)- ausgewählt sind, zusätzlich zu dem Stickstoff, an den R¹³ und R¹⁴ gebunden sind, umfassen, wobei der gesättigte Ring optional 1 oder 2 Kohlenstoff-Kohlenstoff-Doppel- oder -Dreifachbindungen umfasst und die gesättigten und Heteroarylringe optional mit 1 bis 3 R¹⁶-Gruppen substituiert sind;
R¹⁵ H, C₁-C₁₀-Alkyl, C₂-C₁₀-Alkenyl oder C₂-C₁₀-Alkinyl bedeutet, wobei die im vorhergehenden genannten R¹⁵-Gruppen optional mit 1 bis 3 Substituenten substituiert sind, die unabhängig voneinander aus Halogen und -OR⁹ ausgewählt sind;
jedes R¹⁶ unabhängig voneinander aus Halogen, Cyano, Nitro, Trifluormethyl, Azido, -C(O)R¹⁷, -C(O)OR¹⁷, -C(O)OR¹⁷, -OC(O)OR¹⁷, -NR⁶C(O)R⁷, -C(O)NR⁶R⁷, -NR⁶R⁷, Hydroxy, C₁-C₆-Alkyl, C₁-C₆-Alkoxy, -(CH₂)ₘ(C₆-C₁₀-Aryl) und -(CH₂)ₘ(5-10-gliedriges Heteroaryl), wobei m eine ganze Zahl im Bereich von 0 bis 4 ist, ausgewählt ist, und wobei die Aryl- und Heteroarylsubstituenten optional mit 1 oder 2 Substituenten substituiert sind, die unabhängig voneinander aus Halogen, Cyano, Nitro, Trifluormethyl, Azido, -C(O)R¹⁷, -C(O)OR¹⁷, -C(O)OR¹⁷, -OC(O)OR¹⁷, -NR⁶C(O)R⁷, -C(O)NR⁶R⁷, -NR⁶R⁷, Hydroxy, C₁-C₆-Alkyl und C₁-C₆-Alkoxy ausgewählt sind; jedes R¹⁷ unabhängig voneinander aus H, C₁-C₁₀-Alkyl, C₂-C₁₀-Alkenyl, C₂-C₁₀-Alkinyl , -(CH₂)ₘ(C₆-C₁₀-Aryl) und -(CH₂)ₘ(5-10-gliedriges Heteroaryl), wobei m eine ganze Zahl im Bereich von 0 bis 4 ist, ausgewählt ist;
mit dem Vorbehalt, dass R⁸ nicht H ist, wenn R³ -CH₂S(O)ₙR⁸ ist;
wobei das Verfahren die Behandlung einer Verbindung der Formel 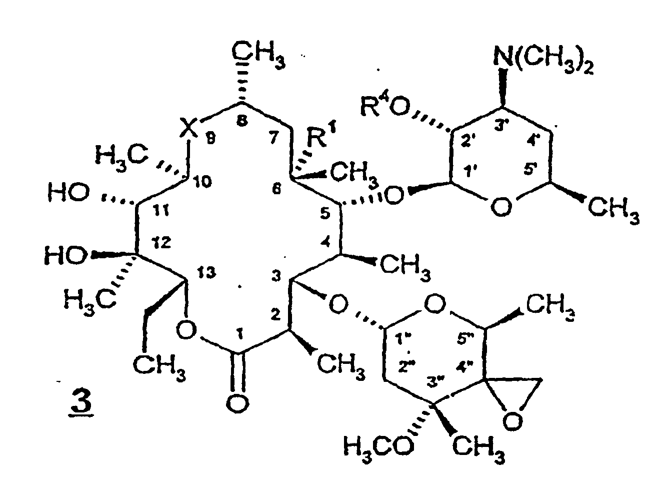
worin X, R¹ und R⁴ wie oben definiert sind, mit einer Verbindung der Formel HOR⁸, HSR⁸ oder HNR¹⁵R⁸, wobei n, R¹⁵ und R⁸ wie oben definiert sind, umfasst, wobei, wenn die Verbindung der Formel HSR⁸ verwendet wird, die gebildete R³-Gruppe der Formel -CH₂SR⁸ optional zu -CH₂S(O)R⁸ oder -CH₂S(O)₂R⁸ oxidiert wird.

27. Verfahren nach Anspruch 26, wobei die Verbindung der Formel 3 durch Behandlung einer Verbindung der Formel 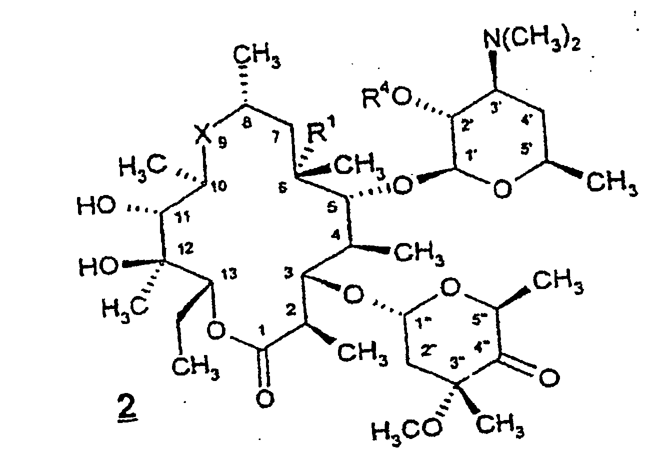 worin X, R¹ und R⁴ wie in Anspruch 24 definiert sind, mit (CH₃)₃S(O)ₙX², wobei n 0 oder 1 ist und X² Halogen, -BF₄ oder -PF₆ ist, in Gegenwart einer Base hergestellt wird.

28. Verfahren nach Anspruch 27 zur Herstellung der Verbindung nach Anspruch 1, wobei X² Iod oder BF₄ ist und die Base aus Kalium-tert-butoxid, Natrium-tert-butoxid, Natriumethoxid, Natriumhydrid, 1,1,3,3-Tetramethylguanidin, 1,8-Diazabicyclo[5.4.0]undec-7-en, 1,5-Diazabicyclo[4.3.0]non-5-en, Kaliumhexamethyldisilazid (KHMDS), Kaliumethoxid und Natriummethoxid ausgewählt ist.

29. Verbindung der Formel 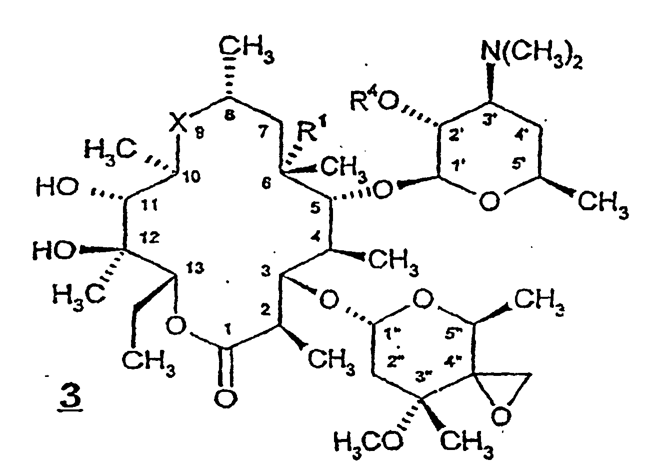 oder ein pharmazeutisch akzeptables Salz derselben, worin
X -CH(NR⁹R¹⁰)-, -C(O)-, -C(=NOR⁹)-, -CH₂NR⁹- oder -N(C₁-C₆-Alkyl)CH₂- bedeutet, wobei der erste Strich von jeder der im vorhergehenden genannten X-Gruppen an den C-10-Kohlenstoff der Verbindung der Formel 3 gebunden ist und der letzte Strich jeder Gruppe an den C-8-Kohlenstoff der Verbindung der Formel 3 gebunden ist; R¹ H, Hydroxy oder Methoxy bedeutet;
R⁴ H, -C(O)R⁹, -C(O)OR⁹, -C(O)NR⁹R¹⁰ oder eine Hydroxyschutzgruppe bedeutet;
jedes R⁹ und R¹⁰ unabhängig voneinander H oder C₁-C₆-Alkyl bedeutet.

## Revendications

1. Composé de formule 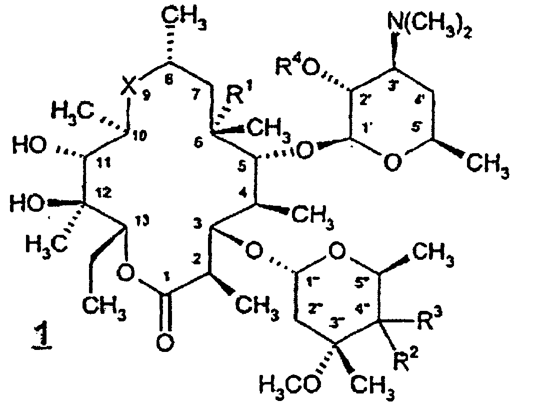 ou un de ses sels pharmaceutiquement acceptables, formule dans laquelle:
X représente un groupe -CH(NR⁹R¹⁰)-, -C(O)-, -C(=NOR⁹)-, -CH₂NR⁹- ou -N(alkyle en C₁ à C₆)CH₂-, dans lequel le premier tiret de chacun des groupes X précités est fixé à l'atome de carbone C-10 du composé de formule 1 et le dernier tiret de chaque groupe est fixé à l'atome de carbone C-8 du composé de formule 1 ;
R¹ représente H, un groupe hydroxy ou méthoxy ;
R² représente un groupe hydroxy ;
R³ représente un groupe alkyle en C₁ à C₁₀, alcényle en C₂ à C₁₀, alcynyle en C₂ à C₁₀, cyano, -CH₂S(O)ₙR⁸ dans lequel n représente un nombre entier de 0 à 2, -CH₂OR⁸, -CH₂N(OR⁹)R⁸, -CH₂NR⁸R¹⁵, -(CH₂)ₘ(aryle en C₆ à C₁₀) ou -(CH₂)ₘ-(hétéroaryle penta- à décagonal), dans lequel m représente un nombre entier de 0 à 4, et les groupes R³ précités sont facultativement substitués avec 1 à 3 groupes R¹⁶ ;
ou bien R² et R³ sont pris conjointement pour former un noyau oxazolyle représenté comme ci-dessous 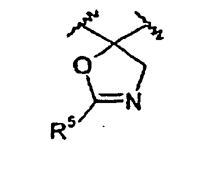 R⁴ représente H, un groupe -C(O)R⁹, -C(O)OR⁹, -C(O)NR⁹R¹⁰ ou un groupe protecteur de la fonction hydroxy ;
R⁵ représente un groupe SR⁸, -(CH₂)ₙC(O)R⁸, dans lequel n est égal à 0 ou 1, alkyle en C₁ à C₁₀, alcényle en C₂ à C₁₀, alcynyle en C₂ à C₁₀, -(CH₂)ₘ(aryle en C₆ à C₁₀) ou -(CH₂)ₘ(hétéroaryle penta- à décagonal) dans lequel m représente un nombre entier de 0 à 4, et les groupes R⁵ précités sont facultativement substitués avec 1 à 3 groupes R¹⁶ ;
chacun des groupes R⁶ et R⁷ représente indépendamment H, un groupe hydroxy, alkoxy en C₁ à C₆, alkyle en C₁ à C₆, alcényle en C₂ à C₆, alcynyle en C₂ à C₆, -(CH₂)ₘ(aryle en C₆ à C₁₀) ou -(CH₂)ₘ(hétéroaryle penta- à décagonal), dans lequel m représente un nombre entier de 0 à 4 ;
chaque groupe R⁸ représente indépendamment H, un groupe alkyle en C₁ à C₁₀, alcényle en C₂ à C₁₀, alcynyle en C₂ à C₁₀, -(CH₂)_{q}CR¹¹R¹²(CH₂)ᵣNR¹³R¹⁴ dans lequel q et r représentent chacun indépendamment un nombre entier de 0 à 3, sauf que q et r ne sont pas l'un et l'autre égaux à 0, -(CH₂)ₘ(aryle en C₆ à C₁₀) ou -(CH₂)ₘ(hétéroaryle penta- à décagonal) dans lequel m représente un nombre entier de 0 à 4, et les groupes R⁸ précités, à l'exception de H, sont facultativement substitués avec 1 à 3 groupes R¹⁶ ;
ou, lorsque R⁸ est présent dans le groupe -CH₂NR⁸R¹⁵, R¹⁵ et R⁸ peuvent être pris conjointement pour former un noyau saturé monocyclique ou polycyclique tétra- à décagonal ou un noyau hétéroaryle penta- à décagonal, lesdits noyaux saturés et noyaux hétéroaryle comprenant facultativement 1 ou 2 hétéroatomes choisis entre O, S et -N(R⁸)-, en plus de l'atome d'azote auquel R¹⁵ et R¹⁸ sont fixés, ledit noyau saturé comprenant facultativement 1 ou 2 doubles ou triples liaisons carbone-carbone, et lesdits noyaux saturés et noyaux hétéroaryle étant facultativement substitués avec 1 à 3 groupes R¹⁶ ;
chacun des groupes R⁹ et R¹⁰ représente indépendamment H ou un groupe alkyle en C₁ à C₆ ;
chacun des groupes R¹¹, R¹², R¹³ et R¹⁴ est choisi indépendamment entre H, des groupes alkyle en C₁ à C₁₀, -(CH₂)ₘ(aryle en C₆ à C₁₀) et -(CH₂)ₘ(hétéroaryle penta- à décagonal), dans lesquels m représente un nombre entier de 0 à 4 et les groupes R¹¹, R¹², R¹³ et R¹⁴ précités, à l'exception de H, sont facultativement substitués avec 1 à 3 groupes R¹⁶ ;
ou bien R¹¹ et R¹³ sont pris conjointement pour former un groupe -(CH₂)ₚ- dans lequel p représente un nombre entier de 0 à 3 de telle sorte que soit formé un noyau saturé tétra- à heptagonal qui comprend facultativement 1 ou 2 doubles ou triples liaisons carbone-carbone ;
ou bien R¹³ et R¹⁴ sont pris conjointement pour former un noyau saturé monocyclique ou polycyclique tétra- à décagonal ou un noyau hétéroaryle penta- à décagonal, lesdits noyaux saturés et noyaux hétéroaryle comprenant facultativement 1 ou 2 hétéroatomes choisis entre O, S et -N(R⁸)-, en plus de l'atome d'azote auquel R¹³ et R¹⁴ sont fixés, ledit noyau saturé comprenant facultativement une ou deux doubles ou triples liaisons carbone-carbone, et lesdits noyaux saturés et noyaux hétéroaryle étant facultativement substitués avec 1 à 3 groupes R¹⁶ ;
R¹⁵ représente H, un groupe alkyle en C₁ à C₁₀, alcényle en C₂ à C₁₀ ou alcynyle en C₂ à C₁₀, les groupes R¹⁵ précités étant facultativement substitués avec 1 à 3 substituants choisis indépendamment entre des substituants halogéno et -OR⁹ ;
chaque groupe R¹⁶ est choisi indépendamment entre des groupes halogéno, cyano, nitro, trifluorométhyle, azido, -C(O)R¹⁷, -C(O)OR¹⁷, -OC(O)OR¹⁷, -NR⁶C(O)R⁷, -C(O)NR⁶R⁷, -NR⁶R⁷, hydroxy, alkyle en C₁ à C₆, alkoxy en C₁ à C₆, -(CH₂)ₘ (aryle en C₆ à C₁₀) et -(CH₂)ₘ(hétéroaryle penta- à décagonal) dans lesquels m représente un nombre entier de 0 à 4, et les substituants aryle et substituants hétéroaryle sont facultativement substitués avec 1 ou 2 substituants choisis indépendamment entre des substituants halogéno, cyano, nitro, trifluorométhyle, azido, -C(O)R¹⁷ -C(O)OR¹⁷, -OC(O)OR¹⁷, -NR⁶C(O)R⁷, -C(O)NR⁶R⁷, -NR⁶R⁷, hydroxy, alkyle en C₁ à C₆ et alkoxy en C₁ à C₆ ;
chaque groupe R¹⁷ est choisi indépendamment entre H, des groupes alkyle en C₁ à C₁₀, alcényle en C₂ à C₁₀, alcynyle en C₂ à C₁₀, -(CH₂)ₘ(aryle en C₆ à C₁₀) et -(CH₂)ₘ(hétéroaryle penta- à décagonal) dans lesquels m représente un nombre entier de 0 à 4 ;
sous réserve que R⁸ ne représente pas H lorsque R³ représente un groupe -CH₂S(O)ₙR⁸.

2. Composé suivant la revendication 1, dans lequel R⁴ représente H, un groupe acétyle ou benzyloxycarbonyle.

3. Composé suivant la revendication 2, dans lequel R¹ représente un groupe hydroxy, R² représente un groupe hydroxy, R³ représente un groupe -CH₂NR¹⁵R⁸ ou -CH₂SR⁸.

4. Composé suivant la revendication 3, dans lequel R³ représente un groupe -CH₂NR¹⁵R⁸ et R¹⁵ et R⁸ sont choisis indépendamment entre H, des groupes alkyle en C₁ à C₁₀, alcényle en C₂ à C₁₀ et alcynyle en C₂ à C₁₀, les groupes R¹⁵ et R⁸ précités, à l'exception de H, étant facultativement substitués avec 1 ou 2 substituants choisis indépendamment entre des substituants hydroxy, halogéno et alkoxy en C₁ à C₆.

5. Composé suivant la revendication 4, dans lequel R¹⁵ et R⁸ sont choisis chacun indépendamment entre H, des groupes méthyle, éthyle, allyle, n-butyle, isobutyle, 2-méthoxyéthyle, cyclopentyle, 3-méthoxypropyle, 3-éthoxypropyle, n-propyle, isopropyle, 2-hydroxyéthyle, cyclopropyle, 2,2,2-trifluoréthyle, 2-propynyle, sec.-butyle, tertiobutyle et n-hexyle.

6. Composé suivant la revendication 2, dans lequel R¹ représente un groupe hydroxy, R² représente un groupe hydroxy, R³ représente un groupe -CH₂NHR⁸ et R⁸ représente un groupe -(CH₂)ₘ(aryle en C₆ à C₁₀) dans lequel m représente un nombre entier de 0 à 4.

7. Composé suivant la revendication 6, dans lequel R⁸ représente un groupe phényle ou benzyle.

8. Composé suivant la revendication 2, dans lequel R¹ représente un groupe hydroxy, R² représente un groupe hydroxy, R³ représente un groupe -CH₂NR¹⁵R⁸, et R¹⁵ et R⁸ sont pris conjointement pour former un noyau saturé tétra- à heptagonal.

9. Composé suivant la revendication 8, dans lequel R¹⁵ et R⁸ sont pris conjointement pour former un noyau pipéridino, triméthylène-imino ou morpholino.

10. Composé suivant la revendication 2, dans lequel R¹ représente un groupe hydroxy, R² représente un groupe hydroxy, R³ représente un groupe -CH₂NR¹⁵R⁸, et R¹⁵ et R⁸ sont pris conjointement pour former un noyau hétéroaryle penta- à décagonal facultativement substitué avec un ou deux groupes alkyle en C₁ à C₆.

11. Composé suivant la revendication 10, dans lequel R¹⁵ et R⁸ sont pris conjointement pour former un noyau pyrrolidino, triazolyle ou imidazolyle dans lequel les groupes hétéroaryle sont facultativement substitués avec 1 ou 2 groupes méthyle.

12. Composé suivant la revendication 2, dans lequel R¹ représente un groupe hydroxy, R² représente un groupe hydroxy, R³ représente un groupe -CH₂SR⁸, et R⁸ est choisi entre des groupes alkyle en C₁ à C₁₀, alcényle en C₂ à C₁₀ et alcynyle en C₂ à C₁₀, lesdits groupes R⁸ étant facultativement substitués avec 1 ou 2 substituants choisis indépendamment entre des substituants hydroxy, halogéno et alkoxy en C₁ à C₆.

13. Composé suivant la revendication 12, dans lequel R⁸ représente un groupe méthyle, éthyle ou 2-hydroxyéthyle.

14. Composé suivant la revendication 2, dans lequel R¹ représente un groupe hydroxy, R² représente un groupe hydroxy et R³ est choisi entre des groupes alkyle en C₁ à C₁₀, alcényle en C₂ à C₁₀ et alcynyle en C₂ à C₁₀, lesdits groupes R³ étant facultativement substitués avec 1 ou 2 substituants choisis indépendamment entre des substituants hydroxy, -C(O)R¹⁷, -NR⁶R⁷, halogéno, cyano, azido, hétéroaryle penta- à décagonal et alkoxy en C₁ à C₆.

15. Composé suivant la revendication 14, dans lequel R³ représente un groupe méthyle, allyle, vinyle, éthynyle, 1-méthyl-1-propényle, 3-méthoxy-1-propynyle, 3-diméthylamino-1-propynyle, 2-pyridyléthynyle, 1-propynyle, 3-hydroxy-1-propynyle, 3-hydroxy-1-propényle, 3-hydroxypropyle, 3-méthoxy-1-propényle, 3-méthoxypropyle, 1-propynyle, n-butyle, éthyle, propyle, 2-hydroxyéthyle, azidométhyle, formylméthyle, 6-cyano-1-pentynyle, 3-diméthylamino-1-propényle ou 3-diméthylaminopropyle.

16. Composé suivant la revendication 2, dans lequel R¹ représente un groupe hydroxy, R² représente un groupe hydroxy et R³ représente un groupe -(CH₂)ₘ(hétéroaryle penta- à décagonal) dans lequel m représente un nombre entier de 0 à 4.

17. Composé suivant la revendication 16, dans lequel R³ représente un groupe 2-thiényle, 2-pyridyle, 1-méthyl-2-imidazolyle, 2-furyle ou 1-méthyl-2-pyrrolyle.

18. Composé suivant la revendication 2, dans lequel R¹ représente un groupe hydroxy, R² représente un groupe hydroxy et R³ représente un groupe -(CH₂)ₘ(aryle en C₆ à C₁₀) dans lequel m représente un nombre entier de 0 à 4.

19. Composé suivant la revendication 18, dans lequel R³ représente un groupe phényle.

20. Composé suivant la revendication 2, dans lequel R² et R³ sont pris conjointement pour former un noyau oxazolyle représenté ci-dessous

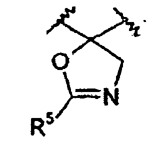

21. Composé suivant la revendication 2, dans lequel R³ est choisi entre des groupes de formule suivante : 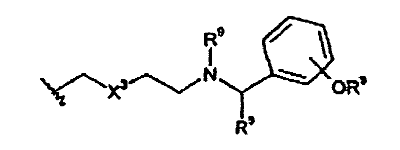 dans laquelle X³ représente O, S ou -N(R¹⁵)-, R⁹ et R¹⁵ répondent aux définitions suivant la revendication 1 et le groupe -OR⁹ peut être fixé à n'importe quel atome de carbone disponible sur le groupe phényle.

22. Composé suivant la revendication 1, destiné à être utilisé comme médicament.

23. Utilisation d'un composé suivant la revendication 1 pour la production d'un médicament destiné au traitement d'une infection bactérienne ou d'une infection par des protozoaires chez un mammifère, un poisson ou un oiseau.

24. Composition comprenant un composé suivant la revendication 1 et un support pharmaceutiquement acceptable.

25. Composition suivant la revendication 24, ladite composition étant destinée au traitement d'une infection bactérienne ou infection par des protozoaires chez un mammifère, un poisson ou un oiseau.

26. Procédé de préparation d'un composé de formule 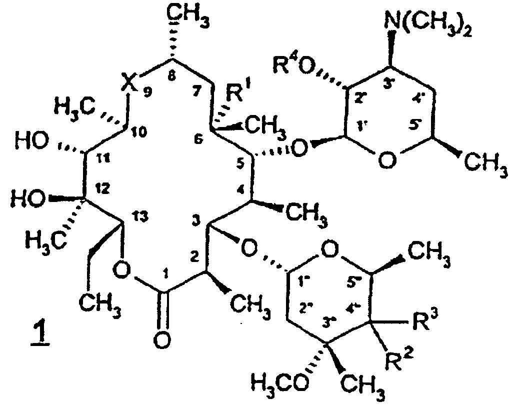 ou d'un de ses sels pharmaceutiquement acceptables, formule dans laquelle:
X représente un groupe -CH(NR⁹R¹⁰)-, -C(O), -C(=NOR⁹)-, -CH₂NR⁹- ou -N(alkyle en C₁ à C₆)CH₂-, dans lequel le premier tiret de chacun des groupes X précités est fixé à l'atome de carbone C-10 du composé de formule 1 et le dernier tiret de chaque groupe est fixé à l'atome de carbone C-8 du composé de formule 1 ;
R¹ représente H, un groupe hydroxy ou méthoxy ;
R² représente un groupe hydroxy ;
R³ représente un groupe alkyle en C₁ à C₁₀, alcényle en C₂ à C₁₀, alcynyle en C₂ à C₁₀, cyano, -CH₂S(O)ₙR⁸ dans lequel n représente un nombre entier de 0 à 2, -CH₂OR⁸, -CH₂N(OR⁹)R⁸, -CH₂NR⁸R¹⁵, (CH₂)ₘ(aryle en C₆ à C₁₀) ou -(CH₂)ₘ(hétéroaryle penta- à décagonal), dans lequel m représente un nombre entier de 0 à 4, et les groupes R³ sont facultativement substitués avec 1 à 3 groupes R¹⁶;
ou bien R² et R³ sont pris conjointement pour former un noyau oxazolyle représenté comme ci-dessous 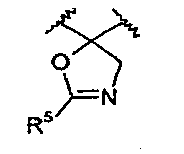
R⁴ représente H, un groupe -C(O)R⁹, -C(O)OR⁹, -C(O)NR⁹R¹⁰ ou un groupe protecteur de la fonction hydroxy ;
R⁵ représente un groupe -SR⁸, -(CH₂)ₙC(O)R⁸, dans lequel n est égal à 0 ou 1, alkyle en C₁ à C₁₀, alcényle en C₂ à C₁₀, alcynyle en C₂ à C₁₀, -(CH₂)ₘ(aryle en C₆ à C₁₀) ou -(CH₂)ₘ(hétéroaryle penta- à décagonal) dans lequel m représente un nombre entier de 0 à 4, et les groupes R⁵ précités sont facultativement substitués avec 1 à 3 groupes R¹⁶ ;
chacun des groupes R⁶ et R⁷ représente indépendamment H, un groupe hydroxy, alkoxy en C₁ à C₆, alkyle en C₁ à C₆, alcényle en C₂ à C₆, alcynyle en C₂ à C₆, -(CH₂)ₘ(aryle en C₆ à C₁₀) ou -(CH₂)ₘ(hétéroaryle penta- à décagonal), dans lequel m représente un nombre entier de 0 à 4 ;
chaque groupe R⁸ représente indépendamment H, un groupe alkyle en C₁ à C₁₀, alcényle en C₂ à C₁₀, alcynyle en C₂ à C₁₀, -(CH₂)_{q}CR¹¹R¹²(CH₂)ᵣNR¹³R¹⁴ dans lequel q et r représentent chacun indépendamment un nombre entier de 0 à 3, sauf que q et r ne sont pas l'un et l'autre égaux à 0, -(CH₂)ₘ(aryle en C₆ à C₁₀) ou -(CH₂)ₘ(hétéroaryle penta- à décagonal) dans lequel m représente un nombre entier de 0 à 4, et les groupes R⁸ précités, à l'exception de H, sont facultativement substitués avec 1 à 3 groupes R¹⁶ ;
ou, lorsque R⁸ représente le groupe -CH₂NR⁸R¹⁵, R¹⁵ et R⁸ peuvent être pris conjointement pour former un noyau saturé monocyclique ou polycyclique tétra- à décagonal ou un noyau hétéroaryle comprenant facultativement 1 ou 2 hétéroatomes choisis entre O, S et -N(R⁸)-, en plus de l'atome d'azote auquel R¹⁵ et R⁸ sont fixés, ledit noyau saturé comprenant facultativement 1 ou 2 doubles ou triples liaisons carbone-carbone, et lesdits noyaux saturés et noyaux hétéroaryle étant facultativement substitués avec 1 à 3 groupes R¹⁶ ;
chacun des groupes R⁹ et R¹⁰ représente indépendamment H ou un groupe alkyle en C₁ à C₆ ;
chacun des groupes R¹¹, R¹², R¹³ et R¹⁴ est choisi indépendamment entre H, des groupes alkyle en C₁ à C₁₀, -(CH₂)ₘ(aryle en C₆ à C₁₀) et -(CH₂)ₘ(hétéroaryle penta- à décagonal), dans lesquels m représente un nombre entier de 0 à 4 et les groupes R¹¹, R¹², R¹³ et R¹⁴ précités, à l'exception de H, sont facultativement substitués avec 1 à 3 groupes R¹⁶ ;
ou bien R¹¹ et R¹³ sont pris conjointement pour former un groupe -(CH₂)ₚ- dans lequel p représente un nombre entier de 0 à 3 de telle sorte que soit formé un noyau saturé tétra- à heptagonal qui comprend facultativement 1 ou 2 doubles ou triples liaisons carbone-carbone ;
ou bien R¹³ et R¹⁴ sont pris conjointement pour former un noyau saturé monocyclique ou polycyclique tétra- à décagonal ou un noyau hétéroaryle penta- à décagonal, lesdits noyaux saturés et noyaux hétéroaryle comprenant facultativement 1 ou 2 hétéroatomes choisis entre O, S et -N(R⁸)-, en plus de l'atome d'azote auquel R¹³ et R¹⁴ sont fixés, ledit noyau saturé comprenant facultativement une ou deux doubles ou triples liaisons carbone-carbone, et lesdits noyaux saturés et noyaux hétéroaryle étant facultativement substitués avec 1 à 3 groupes R¹⁶ ;
R¹⁵ représente H, un groupe alkyle en C₁ à C₁₀, alcényle en C₂ à C₁₀ ou alcynyle en C₂ à C₁₀, les groupes R¹⁵ précités étant facultativement substitués avec 1 à 3 substituants choisis indépendamment entre des substituants halogéno et -OR⁹ ;
chaque groupe R¹⁶ est choisi indépendamment entre des groupes halogéno, cyano, nitro, trifluorométhyle, azido, -C(O)R¹⁷, -C(O)OR¹⁷, -OC(O)OR¹⁷, -NR⁶C(O)R⁷, -C(O)NR⁶R⁷, -NR⁶R⁷, hydroxy, alkyle en C₁ à C₆, alkoxy en C₁ à C₆, - (CH₂)ₘ(aryle en C₆ à C₁₀) et -(CH₂)ₘ(hétéroaryle penta- à décagonal) dans lesquels m représente un nombre entier de 0 à 4, et les substituants aryle et substituants hétéroaryle sont facultativement substitués avec 1 ou 2 substituants choisis indépendamment entre des substituants halogéno, cyano, nitro, trifluorométhyle, azido, -C(O)R¹⁷, -C(O)OR¹⁷, -OC(O)OR¹⁷, -NR⁶C(O)R⁷, -C(O)NR⁶R⁷, -NR⁶R⁷, hydroxy, alkyle en C₁ à C₆ et alkoxy en C₁ à C₆ ;
chaque groupe R¹⁷ est choisi indépendamment entre H, des groupes alkyle en C₁ à C₁₀, alcényle en C₂ à C₁₀, alcynyle en C₂ à C₁₀, -(CH₂)ₘ(aryle en C₆ à C₁₀) et -(CH₂)ₘ(hétéroaryle penta- à décagonal) dans lesquels m représente un nombre entier de 0 à 4 ;
sous réserve que R⁸ ne représente pas H lorsque R³ représente un groupe -CH₂S(O)ₙR⁸ ;
qui comprend le traitement d'un composé de formule 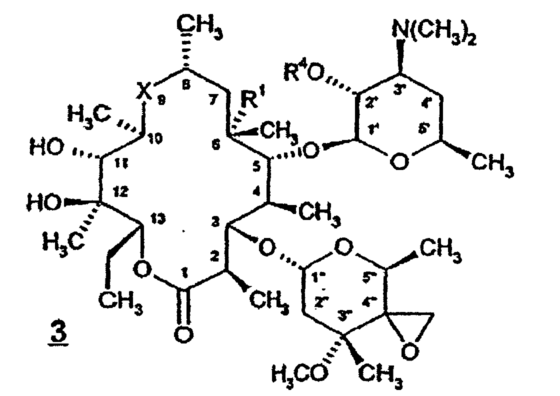
dans laquelle X, R¹ et R⁴ répondent aux définitions précitées, avec un composé de formule HOR⁸, HSR⁸ ou HNR¹⁵R⁸, dans laquelle n, R¹⁵ et R⁸répondent aux définitions précitées, et le groupe R³ résultant de formule -CH₂SR⁸, si ledit composé de formule HSR⁸ est utilisé, étant facultativement oxydé en un groupe -CH₂S(O)R⁸ ou -CH₂S(O)₂R⁸.

27. Procédé suivant la revendication 26, dans lequel le composé de formule 3 est préparé en traitant un composé de formule 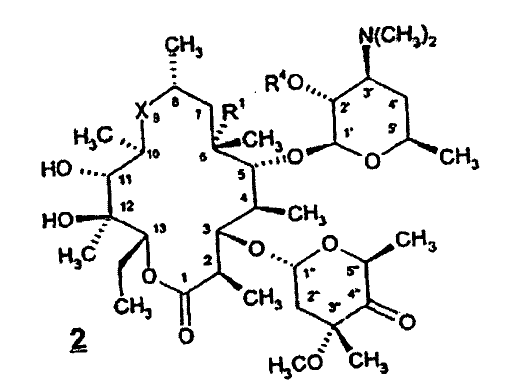 dans laquelle X, R¹ et R⁴ répondent aux définitions suivant la revendication 24, avec un composé de formule (CH₃)₃S(O)ₙX², dans laquelle n est égal à 0 ou 1 et X² représente un groupe halogéno, -BF₄ ou -PF₆, en présence d'une base.

28. Procédé suivant la revendication 27 pour la production du composé de la revendication 1, dans lequel X² représente un groupe iodo ou BF₄ et ladite base est choisie entre le tertiobutylate de potassium, le tertiobutylate de sodium, l'éthylate de sodium, l'hydrure de sodium, la 1,1,3,3-tétraméthylguanidine, le 1,8-diazabicyclo[5.4.0]-undéc-7-ène, le 1,5-diazabicylo[4.3.0]-none-5-ène, l'hexaméthyldisilazoture de potassium (KHMDS), l'éthylate de potassium et le méthylate de sodium.

29. Composé de formule 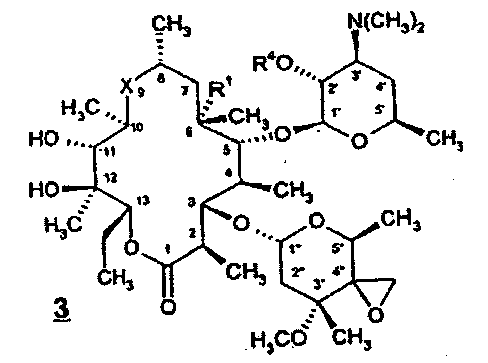 ou un de ses sels pharmaceutiquement acceptables, formule dans laquelle:
X représente un groupe -CH(NR⁹R¹⁰)-, -C(O)-, -C(=NOR⁹)-, -CH₂NR⁹- ou -N(alkyle en C₁ à C₆)CH₂- dans lequel le premier tiret de chacun des groupes X précités est fixé à l'atome de carbone C-10 du composé de formule 3 et le dernier tiret de chaque groupe est fixé à l'atome de carbone C-8 du composé de formule 3 ;
R¹ représente H, un groupe hydroxy ou méthoxy ;
R⁴ représente H, un groupe -C(O)R⁹, -C(O)OR⁹, -C(O)NR⁹R¹⁰ ou un groupe protecteur de la fonction hydroxy ;
chacun des groupes R⁹ et R¹⁰ représenté indépendamment H ou un groupe alkyle en C₁ à C₆.
